# EUROPÄISCHE PATENTSCHRIFT

(11) **EP 2 947 078 B1**
(45) Veröffentlichungstag und Bekanntmachung des Hinweises auf die Patenterteilung: **21.03.2018**
(21) Anmeldenummer: 14169354.9
(22) Anmeldetag: 21.05.2014
(51) Int. Cl.: C07D 317/70, C11B 9/00

(54) **Neue Mischungen mit (4aR,5R,7aS,9R)-Octahydro-2,2,5,8,8,9a-hexamethyl-4H-4a,9-Methanoazuleno(5,6-d)-1,3-dioxol) (Ambrocenide®)**
Novel mixtures with (4aR, 5R, 7aS, 9R) -octahydro-2,2,5,8,8,9a-hexamethyl-4h-4a, 9-methanoazuleno (5,6-d) -1,3-dioxol) (ambrocenide ®)
Nouveaux mélanges contenant du (4aR,5R,7aS,9R)-octahydro-2,2,5,8,8,9a-hexaméthylène-4H-4a,9-méthanoazuléno(5,6-d)-1,3-dioxole) (Ambrocenide®)

(43) Veröffentlichungstag der Anmeldung: 25.11.2015
(73) Patentinhaber: Symrise AG, 37603 Holzminden (DE)
(72) Erfinder: Schatkowski, Dietmar, 37574 Einbeck (DE); Knoop, Daniela, 37603 Holzminden (DE); Lambrecht, Stefan, 37619 Hehlen (DE)
(74) Vertreter: Eisenführ Speiser

(56) Entgegenhaltungen:
- EP-A1- 0 857 723
- EP-A2- 1 634 864
- PANTEN J ET AL: "New woody and ambery notes from cedarwood and turpentine oil", CHEMISTRY & BIODIVERSITY, HELVETICA CHIMICA ACTA, ZUERICH, CH, Bd. 1, Nr. 12, 1. Dezember 2004 (2004-12-01), Seiten 1936-1948, XP002543298, ISSN: 1612-1872, DOI: 10.1002/CBDV.200490148

## Beschreibung

Die vorliegende Erfindung betrifft eine Mischung, enthaltend oder zumindest im Wesentlichen bestehend aus Verbindung der Formel (Ia) und Verbindung der Formel (Ib), wobei das Gewichtsverhältnis von der Verbindung der Formel (Ia) zur Verbindung der Formel (Ib) in der Mischung im Bereich von 90 : 10 bis 99 : 1 liegt. Sie betrifft weiterhin ein Verfahren zur Herstellung einer Mischung, enthaltend oder zumindest im Wesentlichen bestehend aus Verbindung der Formel (Ia) und Verbindung der Formel (Ib), wobei das Gewichtsverhältnis von der Verbindung der Formel (Ia) zur Verbindung der Formel (Ib) in der Mischung im Bereich von 90 : 10 bis 99 : 1 liegt umfassend die folgenden Schritte: a) Bereitstellen einer Isomerenmischung, enthaltend oder zumindest im Wesentlichen bestehend aus Verbindungen der Formel (III) (Cedrandiol), wobei die Isomerenmischung alpha,alpha-Cedrandiol der Formel (IIIa) und beta,beta-Cedrandiol der Formel (IIIb) enthält oder zumindest im Wesentlichen daraus besteht und wobei das Gewichtsverhältnis von der Verbindung der Formel (IIIa) zur Verbindung der Formel (IIIb) in der Isomerenmischung im Bereich von 95 : 5 bis 99,9 : 0,1 liegt, b) Umsetzen der Isomerenmischung aus Schritt a) mit Dimethoxypropan im Molverhältnis von wenigstens 1 : 2, bezogen auf die Gesamt-Molmenge an Verbindungen der Formel (III) zur Gesamt-Molmenge an Dimethoxypropan. Sie betrifft ferner eine Riech- und/oder Aromastoffkomposition, enthaltend oder bestehend aus einer erfindungsgemäßen Mischung sowie ein parfümiertes Produkt, enthaltend eine erfindungsgemäße Mischung in einer sensorisch wirksamen Menge. Sie betrifft außerdem die Verwendung einer erfindungsgemäßen Mischung (a) zum Maskieren oder Vermindern des bzw. eines oder mehrerer unangenehmer Geruchseindrücke eines oder mehrerer unangenehm riechender Stoffe, und/oder (b) zum Verstärken des bzw. eines oder mehrerer angenehmer Geruchseindrücke eines oder mehrerer angenehm riechender Stoffe.

EP 0 857 723 A1 beschreibt eine Reihe von cyclischen Cedren-Acetalen, Verfahren zu deren Herstellung sowie die Verwendung solcher Verbindungen in Riechstoffmischungen, bzw. Parfümölen, zum Verstärken unterschiedlicher Parfümnoten und zum Verlängern der Duftwirkung.

In "New Woody and Ambery Notes from Cedarwood and Turpentine Oil" (Panten et al., Chemistry and Biodiversity, 2004, Vol. 1, 1936-1948) wird offenbart, dass Longifolen aus Terpentinöl und Cedren aus Zedernholz gängige Ausgangsmaterialien für die Herstellung von Duftstoffen darstellen. Ambrocenide®, welches in drei Syntheseschritten aus alpha-Cedren erhalten werden kann, ist die geeignetste Verbindung für Holz- und Amber-Noten.

Ambrocenide® besitzt die folgende chemische Struktur (Abb. 1):

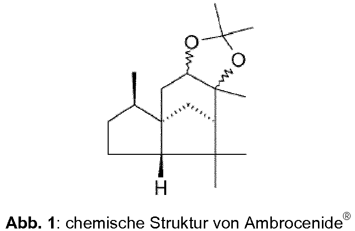

Die geschlängelten Linien können dabei unabhängig voneinander alpha- oder beta-Konfiguration bedeuten. Ambrocenide® kann ein, zwei, drei oder sämtliche der folgenden Diastereoisomere umfassen (Abb. 2):

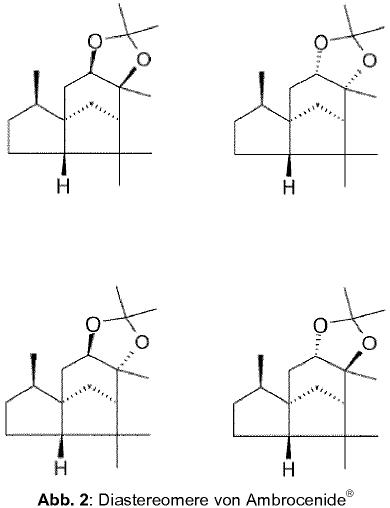

Dementsprechend kann Ambrocenide® isomerenrein als Verbindung der Formel in Abb. 2, oben links, als (Verbindung der Formel in Abb. 2, oben rechts, als Verbindung der Formel in Abb. 2, unten links, als Verbindung der Formel in Abb. 2, unten rechts oder aber als Gemisch zweier, dreier oder aller genannten Stereoisomere vorliegen.

Eine Möglichkeit zur Herstellung von Ambrocenide® besteht darin, zunächst (-)-alpha-Cedren **(1)** durch Behandeln mit Peressigsäure zu (-)-alpha-Cedrenepoxid (**2**) umzusetzen. Das aus Cedren erhaltene Epoxid (**2**) wird dann durch Säure katalysierte Ringöffnung in ein Gemisch der epimeren Cedran-Diole (**3**) übergeführt. Ambrocenide® (**4**, R=R'=CH₃) wird aus den Diolen (**3**) durch Umsetzung mit Dimethoxypropan unter Säurekatalyse erhalten (Abb. 3).

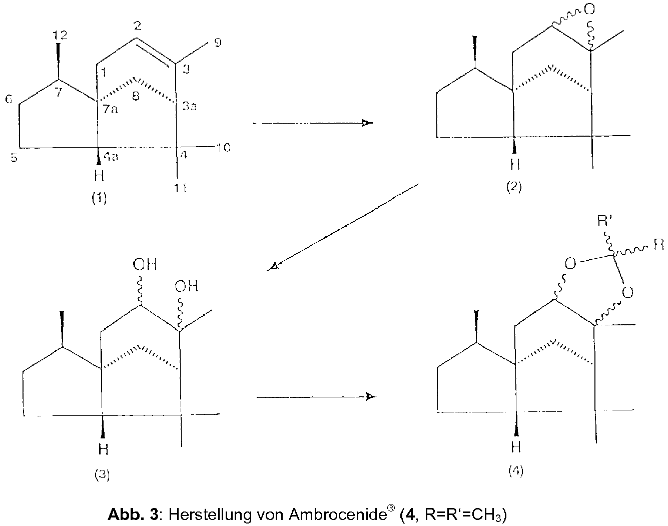

Die epimerenreinen Epoxide (**2a**) und (**2b**) lassen sich einzeln oder im Gemisch in die diastereomeren Cedran-Diolen (**3a-h**) überführen. In Abhängigkeit von den gewählten Reaktionsbedingungen bei der Umsetzung von (-)-a-Cedren **(1)** mit Peressigsäure können die epimeren Epoxide (**2a**) und (**2b**) in unterschiedlichen Mengenverhältnissen anfallen, so dass nach Öffnung der Epoxide zu den diastereomeren Diolen (**3a-h**) diese ebenfalls in unterschiedlichen Mengenverhältnissen vorliegen (Abb. 4).

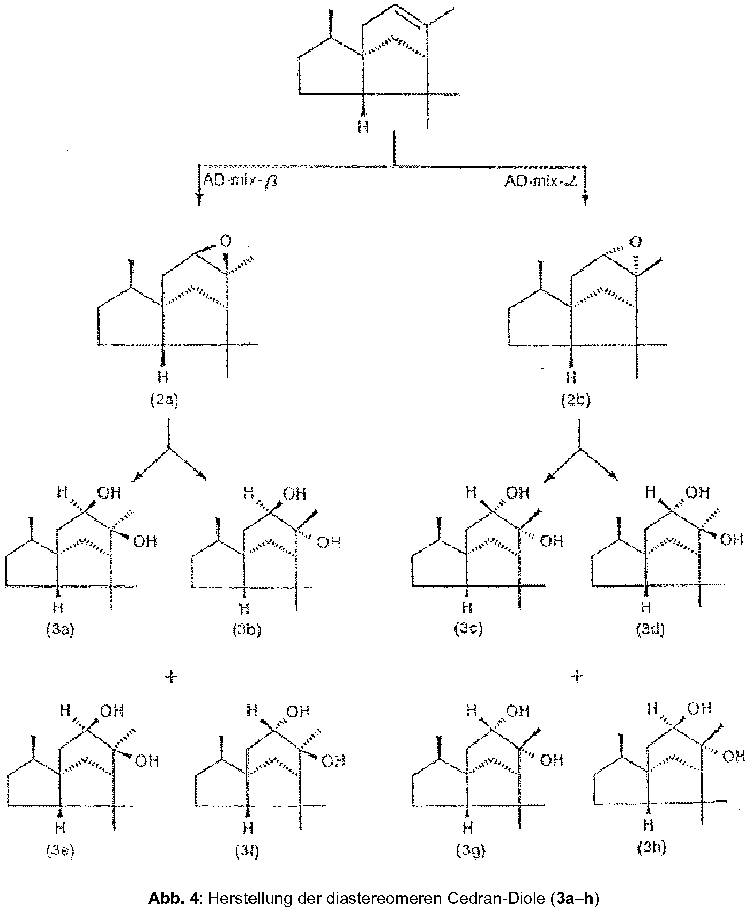

Die stereochemischen Verhältnisse bleiben bei der nachfolgenden Umsetzung der Cedran-Diole mit Dimethoxypropan im Wesentlichen unverändert, so dass Ambrocenide® ebenfalls als Diastereomerengemisch vorliegt.

Bisher ist Ambrocenide® nicht in kristalliner Form, sondern nur als Lösung, beispielsweise als 10 Gew.-%ige Lösung in Dipropylenglycol (Ambrocenide® 10DPG) erhältlich. Eine Aufgabe der vorliegenden Erfindung ist es daher, Ambrocenide® in im Wesentlichen kristalliner Form bereitzustellen. Unter einer "im Wesentlichen kristallinen Form" wird gemäß der vorliegenden Erfindung verstanden, dass wenigstens 90 Gew.-% bezogen auf die Gesamtmenge an Ambrocenide® in kristalliner Form vorliegen.

Diese Aufgabe wird erfindungsgemäß in einem ersten Aspekt gelöst durch eine Mischung, enthaltend oder zumindest im Wesentlichen bestehend aus
- Verbindung der Formel (Ia) 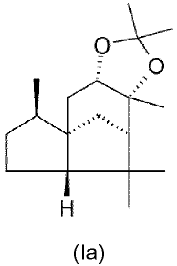 und
- Verbindung der Formel (Ib), 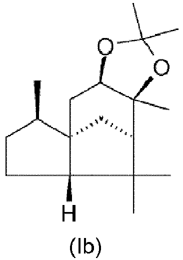
wobei das Gewichtsverhältnis von der Verbindung der Formel (Ia) zur Verbindung der Formel (Ib) in der Mischung im Bereich von 90 : 10 bis 99 : 1 liegt.

Unter "im Wesentlichen bestehend aus" wird gemäß der vorliegenden Erfindung verstanden, dass die erfindungsgemäße Mischung zu mindestens 85 Gew.-%, bevorzugt zu mindestens 90 Gew.-%, besonders bevorzugt zu mindestens 95 Gew.-%, ganz besonders bevorzugt zu mindestens 98 Gew.-% aus Verbindungen der Formeln (Ia) und (Ib) besteht.

In einer bevorzugten Ausgestaltung betriff die vorliegende Erfindung eine Mischung wie hierin beschrieben, wobei das Gewichtsverhältnis von der Verbindung der Formel (Ia) zur Verbindung der Formel (Ib) in der Mischung im Bereich von 95 : 5 bis 99 : 1, vorzugsweise bei etwa 98 : 2, liegt.

Ferner betrifft die vorliegende Erfindung in einer weiteren bevorzugten Ausgestaltung eine Mischung wie hierin beschrieben, wobei die Mischung zudem eine Verbindung der Formel (II) (Cedralon) enthält, 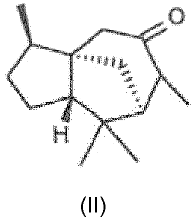 wobei das Gewichtsverhältnis der Gesamtmenge an Verbindungen der Formeln (Ia) und (Ib) zur Gesamtmenge an Verbindung der Formel (II) in der Mischung wenigstens 6 : 1, vorzugsweise wenigstens 9 : 1, weiter bevorzugt wenigstens 12 : 1, besonders bevorzugt wenigstens 25 : 1, ist.

Ebenfalls betrifft die vorliegende Erfindung in einer weiteren bevorzugten Ausgestaltung eine Mischung wie hierin beschrieben, wobei die Mischung zudem eine Verbindung der Formel (III) (Cedrandiol) enthält, 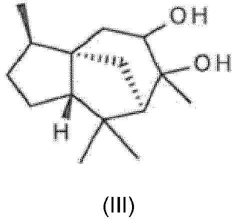 wobei das Gewichtsverhältnis der Gesamtmenge an Verbindungen der Formeln (Ia) und (Ib) zur Gesamtmenge an Verbindung der Formel (III) in der Mischung wenigstens 15 : 1, vorzugsweise wenigstens 20 : 1, besonders bevorzugt wenigstens 25 : 1, ist.

Durch die vorliegende Erfindung ist es vorteilhafterweise möglich, Ambrocenide® in im Wesentlichen kristalliner Form bereitzustellen.

Dementsprechend betrifft die vorliegende Erfindung in einer bevorzugten Ausgestaltung eine erfindungsgemäße Mischung wie hierin beschrieben, wobei wenigstens 90 Gew.-% der Gesamtmenge an Verbindungen der Formeln (Ia) und (Ib) in der Mischung in kristalliner Form vorliegen.

Die hierin beschriebene Mischung ist vorzugsweise herstellbar durch ein Verfahren umfassend die nachfolgenden Schritte:
a) Bereitstellen einer Isomerenmischung, enthaltend oder zumindest im Wesentlichen bestehend aus Verbindungen der Formel (III) (Cedrandiol), wobei die Isomerenmischung alpha,alpha-Cedrandiol der Formel (IIIa) 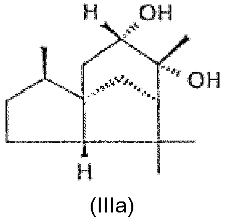 und beta,beta-Cedrandiol der Formel (IIIb) 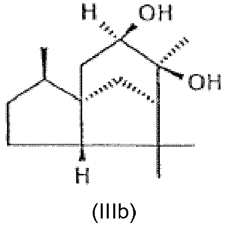 enthält oder zumindest im Wesentlichen daraus besteht, und wobei das Gewichtsverhältnis von der Verbindung der Formel (IIIa) zur Verbindung der Formel (IIIb) in der Isomerenmischung im Bereich von 95 : 5 bis 99,9 : 0,1 liegt, vorzugsweise wenigstens 98 : 2 beträgt,
b) Umsetzen der Isomerenmischung aus Schritt a) mit Dimethoxypropan im Molverhältnis von wenigstens 1 : 2, bezogen auf die Gesamt-Molmenge an Verbindungen der Formel (III) zur Gesamt-Molmenge an Dimethoxypropan, und optional
c) Auskristallisieren des Reaktionsprodukts aus Schritt b) aus wässriger alkoholischer Lösung.

Bezüglich des oben genannten Schrittes b) ist ein Umsetzen der Isomerenmischung aus Schritt a) mit Dimethoxypropan im Molverhältnis im Bereich von etwa 1 : 1 bis etwa 1 : 2, insbesondere von etwa 1 : 2, bezogen auf die Gesamt-Molmenge an Verbindungen der Formel (III) zur Gesamt-Molmenge an Dimethoxypropan, bevorzugt.

Dementsprechend betrifft die vorliegende Erfindung in einem weiteren Aspekt auch ein Verfahren zur Herstellung einer Mischung, enthaltend oder zumindest im Wesentlichen bestehend aus
- Verbindung der Formel (Ia) 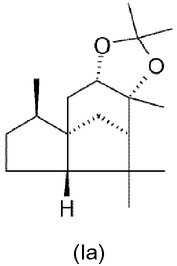 und
- Verbindung der Formel (Ib), 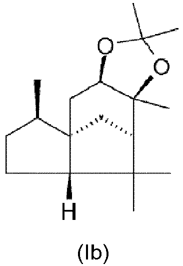 wobei das Gewichtsverhältnis von der Verbindung der Formel (Ia) zur Verbindung der Formel (Ib) in der Mischung im Bereich von 90 : 10 bis 99 : 1 liegt,
   vorzugsweise zur Herstellung einer Mischung wie hierin beschrieben,
   umfassend die folgenden Schritte:
   a) Bereitstellen einer Isomerenmischung, enthaltend oder zumindest im Wesentlichen bestehend aus Verbindungen der Formel (III) (Cedrandiol), wobei die Isomerenmischung alpha,alpha-Cedrandiol der Formel (IIIa) 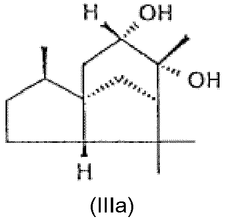 und beta,beta-Cedrandiol der Formel (IIIb) 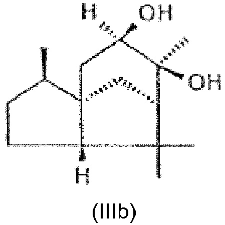 enthält oder zumindest im Wesentlichen daraus besteht und wobei das Gewichtsverhältnis von der Verbindung der Formel (IIIa) zur Verbindung der Formel (IIIb) in der Isomerenmischung im Bereich von 95 : 5 bis 99,9 : 0,1 liegt, vorzugsweise wenigstens 98 : 2 beträgt,
   b) Umsetzen der Isomerenmischung aus Schritt a) mit Dimethoxypropan im Molverhältnis von wenigstens 1 : 2, bezogen auf die Gesamt-Molmenge an Verbindungen der Formel (III) zur Gesamt-Molmenge an Dimethoxypropan,
   optional umfassend den zusätzlichen Schritt
   c) Auskristallisieren des Reaktionsprodukts aus Schritt b) aus wässriger alkoholischer Lösung.

Für ein solches erfindungsgemäßes Verfahren gilt vorzugsweise das oben im Zusammenhang mit einer erfindungsgemäßen Mischung, insbesondere einer bevorzugten Ausgestaltung davon, Gesagte entsprechend.

Die Isomerenmischung, enthaltend oder zumindest im Wesentlichen bestehend aus Verbindungen der Formel (III) (Cedrandiol), wobei die Isomerenmischung alpha,alpha-Cedrandiol der Formel (IIIa) und beta,beta-Cedrandiol der Formel (IIIb) enthält oder zumindest im Wesentlichen daraus besteht und wobei das Gewichtsverhältnis von der Verbindung der Formel (IIIa) zur Verbindung der Formel (IIIb) in der Isomerenmischung im Bereich von 95 : 5 bis 99,9 : 0,1 liegt, vorzugsweise wenigstens 98 : 2 beträgt, ist kommerziell bspw. von der Firma "Bio-Young Aromas Co., Ltd." erhältlich, kann aber beispielsweise auch auf nachfolgendem Weg erhalten werden:
Eine geeignete Menge, beispielsweise 100 g, an Cedrendiolgemisch (bevorzugter Gehalt an cis-Diol: 85 Gew.-%) werden unter Rühren zu einer geeigneten Menge, beispielsweise 400 g, eines Gemisches aus Ethanol/Wasser im bevorzugten Gewichtsverhältnis 4:1 gegeben. Die erhaltene Reaktionsmischung wird vorzugsweise auf 75 °C erwärmt. Bei dieser Temperatur löst sich das Cedrendiol-Gemisch vollständig auf. Die Reaktionsmischung wird dann vorzugsweise bei 75 °C noch 30 Minuten gerührt und anschließend über einen Zeitraum von vorzugsweise 2 Stunden auf 25 °C gekühlt. Der ausfallende kristalline Feststoff wird über eine Filternutsche abgesaugt und getrocknet. Unter Verwendung der vorstehend genannten Mengen kann eine Ausbeute von 70g (in Form weißer Kristalle) erhalten werden. Eine exemplarisch durchgeführte Analytik (GC: 60 °C - 240 °C; Aufheizrate 8 °C pro Minute, Säulentyp DB 1) ergab 0,8 Gew.-% trans-Cedrendiol, 98,5 Gew.-% cis-Cedrendiol und 0,4 Gew.-% Cedralon.

In einem weiteren Aspekt betrifft die vorliegende Erfindung eine Riech- und/oder Aromastoffkomposition, vorzugsweise ein Parfümöl, enthaltend oder bestehend aus einer Mischung wie hierin beschrieben und zudem vorzugsweise einem oder mehreren zusätzlichen Riech- und/oder Aromastoffen.

Ebenfalls betrifft die vorliegende Erfindung in einer bevorzugten Ausgestaltung eine Riech- und/oder Aromastoffkomposition wie hierin beschrieben, wobei der zusätzliche bzw. einer, mehrere oder sämtliche der zusätzlichen Riechstoffe ausgewählt ist bzw. sind aus der Gruppe bestehend aus 3-(4-Methyl-1-cyclohex-3-enyl)-butanal, 4-(4-Hydroxyphenyl) butan-2-on, (E)-4-(2,6,6-Trimethyl-1-cyclohex-2-enyl)but-3-en-2-on, (E)-4-(2,6,6-Trimethyl-1-cyclohex-2-enyl)but-3-en-2-on, (E)-1-(2,6,6-trimethyl-cyclohexen-1-yl)pent-1-en-3-on, (E)-4-[(1S)-1,2,6,6-tetramethylcyclohex-2-en-1-yl]-but-3-en-2-on, 1-(2,6,6-Trimethyl-1-cyclohex-2-enyl)pent-1-en-3-on, [(Z)-Hex-3-enyl] methyl carbonat, 3-[(Z)-Hex-3-enoxy]propannitril, 1-(2,3,8,8-Tetramethyl-1,3,4,5,6,7-hexa-hydronaphthalin-2-yl)ethanon, spiro[1,3-dioxolan-2,5'-(4',4',8',8'-tetramethyl-hexahydro-3',9'-methano-naphthalin)], [3R-(3α,3aβ,6β,7β,8aα)]-octa-hydro-6-methoxy-3,6,8,8-tetramethyl-1H-3a,7-methanoazulen,[3R-(3α,3aβ,7β,8aα)]-1-(2,3,4,7,8,8a-hexahydro-3,6,8,8-tetramethyl-1H-3a,7-methano-azulen-5-yl)ethan-1-on, 1-(2,2,6-Trimethyl-cyclohexyl)hexan-3-ol, 6,6-Dimethoxy-2,5,5-trimethylhex-2-en, 2,6-Dimethyloct-7-en-2-ol, 3,7-Dimethylocta-1,6-dien-3-ol, 3,7-Dimethylocta-1,6-dien-3-yl acetat, (4-Methyl-1-propan-2-yl-1-cyclohex-2-enyl) acetat, (8E)-Cyclohexadec-8-en-1-on, 16-Oxacyclohexa-decan-1-on, 1,3,4,6,7,8-Hexahydro-4,6,6,7,8,8-hexamethyl-cyclopenta(g)-2-benzopyran, Ethoxymethoxy-cyclododecan, 1,1,2,3,3-Pentamethyl-2,5,6,7-tetrahydroinden-4-on, 1-(2,3,8,8-Tetramethyl-1,3,4,5,6,7-hexa-hydronaphthalin-2-yl)ethanon.

Ebenso betrifft die vorliegende Erfindung in einer weiteren bevorzugten Ausgestaltung auch eine Riech- und/oder Aromastoffkomposition wie hierin beschrieben, wobei die Menge an der hierin beschriebenen Mischung bzw. die Menge an Verbindung der Formel (Ia) und Verbindung der Formel (Ib) ausreicht,
(a) um den bzw. einen oder mehrere unangenehme Geruchseindrücke eines anderen Riech- und/oder Aromastoffs in der Riech- und/oder Aromastoffkomposition zu maskieren oder zu vermindern,
   und/oder
(b) um den bzw. einen oder mehrere angenehme Geruchseindrücke eines anderen Riech- und/oder Aromastoffs in der Riech- und/oder Aromastoffkomposition zu verstärken.

Die vorliegende Erfindung betrifft in einer weiteren bevorzugten Ausgestaltung eine Riech- und/oder Aromastoffkomposition wie hierin beschrieben, vorzugsweise ein Parfümöl, wobei die Gesamtmenge an Verbindung der Formel (Ia) und Verbindung der Formel (Ib) bezogen auf das Gesamtgewicht der Riech- und/oder Aromastoffkomposition 0,01 bis 10 Gew.-%, vorzugsweise 0,03 bis 5 Gew.-%, besonders bevorzugt 0,05 bis 2 Gew.-%, beträgt.

In einem weiteren Aspekt betrifft die vorliegende Erfindung ein parfümiertes Produkt, enthaltend eine Mischung wie hierin beschrieben oder, vorzugsweise, eine Riech- und/oder Aromastoffkomposition wie hierin beschrieben, vorzugsweise ein Parfümöl, in einer sensorisch wirksamen Menge, wobei der Anteil der Mischung bzw. der Riech- und/oder Aromastoffkomposition bezogen auf das Gesamtgewicht des Produkts vorzugsweise im Bereich von 0,01 bis 10 Gew.-%, bevorzugt im Bereich von 0,1 bis 5 Gew.-%, besonders bevorzugt im Bereich von 0,25 bis 3 Gew.-%, liegt.

Ebenfalls betrifft die vorliegende Erfindung in einer bevorzugten Ausgestaltung ein parfümiertes Produkt wie hierin beschrieben, wobei das Produkt ausgewählt ist aus der Gruppe bestehend aus Parfüm-Extraits, Eau de Parfums, Eau de Toilettes, Rasierwässer, Eau de Colognes, Pre-shave-Produkte, Splash-Colognes, parfümierte Erfrischungstücher, saure, alkalische und neutrale Reinigungsmittel, Textilerfrischer, Bügelhilfen, flüssige Waschmittel, pulverförmige Waschmittel, Wäschevorbehandlungsmittel, Wäscheweichspüler, Waschseifen, Waschtabletten, Desinfektionsmittel, Oberflächendesinfektionsmittel, Luftverbesserer, Aerosolsprays, Wachse und Polituren, Körperpflegemittel, Handcremes und -lotionen, Fußcremes und - lotionen, Enthaarungscremes und -lotionen, After-shave-Cremes und -lotionen, Bräunungscremes und -lotionen, Haarpflegeprodukten, Deodorantien und Antiperspirantien, Produkte der dekorativen Kosmetik, Kerzen, Lampenöle, Räucherstäbchen, Insektizide, Repellentien und Treibstoffe.

In einem weiteren Aspekt betrifft die vorliegende Erfindung ein Verfahren zum Herstellen eines parfümierten Produkts, insbesondere eines parfümierten Produkts wie hierin beschrieben, umfassend folgende Schritte:
i) Bereitstellen einer Mischung nach einem der Ansprüche 1 bis 7 oder einer Riech- und/oder Aromastoffkomposition nach einem der Ansprüche 10 bis 13 oder der Substanzen der Formel (Ia) 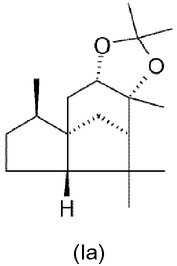 und der Formel (Ib), 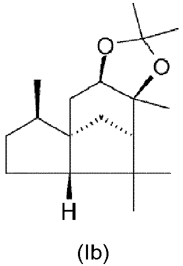 in einem Gewichtsverhältnis wie in Anspruch 1 oder 2 definiert,
ii) Bereitstellen eines oder mehrerer weiterer Bestandteile des herzustellenden parfümierten Produkts, und
iii) Inkontaktbringen oder Mischen der in Schritt ii) bereitgestellten weiteren Bestandteile mit einer sensorisch wirksamen Menge der in Schritt i) bereitgestellten Bestandteile.

In einem weiteren Aspekt betrifft die vorliegende Erfindung die Verwendung einer Mischung wie hierin beschrieben
(a) zum Maskieren oder Vermindern des bzw. eines oder mehrerer unangenehmer Geruchseindrücke eines oder mehrerer unangenehm riechender Stoffe,
   und/oder
(b) zum Verstärken des bzw. eines oder mehrerer angenehmer Geruchseindrücke eines oder mehrerer angenehm riechender Stoffe.

Ebenso betrifft die vorliegende Erfindung in einer bevorzugten Ausgestaltung die Verwendung wie hierin beschrieben in einer Zusammensetzung, vorzugsweise einem Parfümöl, die bzw. das einen oder mehrere (weitere) angenehm und/oder unangenehm riechende Stoffe enthält, deren unangenehmer Geruchseindruck durch die Mischung wie hierin beschrieben maskiert oder vermindert und/oder deren angenehmer Geruchseindruck durch die Mischung wie hierin beschrieben verstärkt wird, wobei diese(r) angenehm und/oder unangenehm riechende Stoff bzw. einer, mehrere oder sämtliche dieser angenehm und/oder unangenehm riechende Stoffe ausgewählt ist bzw. sind aus der Gruppe bestehend aus 3-(4-Methyl-1-cyclohex-3-enyl)-butanal, 4-(4-Hydroxyphenyl) butan-2-on, (E)-4-(2,6,6-Trimethyl-1-cyclohex-2-enyl)but-3-en-2-on, (E)-4-(2,6,6-Trimethyl-1-cyclohex-2-enyl)but-3-en-2-on, (E)-1-(2,6,6-trimethyl-cyclohexen-1-yl)pent-1-en-3-on, (E)-4-[(1S)-1,2,6,6-tetramethylcyclohex-2-en-1-yl]-but-3-en-2-on, 1-(2,6,6-Trimethyl-1-cyclohex-2-enyl)pent-1-en-3-on, [(Z)-Hex-3-enyl] methyl carbonat, 3-[(Z)-Hex-3-enoxy]propannitril, 1-(2,3,8,8-Tetramethyl-1,3,4,5,6,7-hexa-hydronaphthalin-2-yl)ethanon, spiro[1,3-dioxolan-2,5'-(4',4',8',8'-tetramethyl-hexahydro-3',9'-methano-naphthalin)], [3R-(3α,3aβ,6β,7β,8aα)]-octa-hydro-6-methoxy-3,6,8,8-tetramethyl-1H-3a,7-methanoazulen,[3R-(3α,3aβ,7β,8aα)]-1-(2,3,4,7,8,8a-hexahydro-3,6,8,8-tetramethyl-1H-3a,7-methano-azulen-5-yl)ethan-1-on, 1-(2,2,6-Trimethyl-cyclohexyl)hexan-3-ol, 6,6-Dimethoxy-2,5,5-trimethylhex-2-en, 2,6-Dimethyloct-7-en-2-ol, 3,7-Dimethylocta-1,6-dien-3-ol, (3,7-Dimethylocta-1,6-dien-3-yl)acetat, (4-Methyl-1-propan-2-yl-1-cyclohex-2-enyl) acetat, (8E)-Cyclohexadec-8-en-1-on, 16-Oxacyclohexa-decan-1-on, 1,3,4,6,7,8-Hexahydro-4,6,6,7,8,8-hexamethyl-cyclopenta(g)-2-benzopyran, Ethoxymethoxy-cyclododecan, 1,1,2,3,3-Pentamethyl-2,5,6,7-tetrahydroinden-4-on, 1-(2,3,8,8-Tetramethyl-1,3,4,5,6,7-hexa-hydronaphthalin-2-yl)ethanon.

Ebenfalls betrifft die vorliegende Erfindung in einer bevorzugten Ausgestaltung die Verwendung wie hierin beschrieben, zum Verstärken des bzw. eines oder mehrerer Geruchseindrücke ausgewählt aus der Gruppe bestehend aus den Geruchsnoten blumig, Amber, holzig, Moschus, Veilchen, Citrus und aldehydig.

In einem weiteren Aspekt betrifft die vorliegende Erfindung ein Verfahren
(a) zum Maskieren oder Vermindern des bzw. eines oder mehrerer unangenehmer Geruchseindrücke eines oder mehrerer unangenehm riechender Stoffe,
   und/oder
(b) zum Verstärken des bzw. eines oder mehrerer angenehmer Geruchseindrücke eines oder mehrerer angenehm riechender Stoffe,
umfassend folgenden Schritt:

Vermischen des bzw. der (a) angenehm und/oder (b) unangenehm riechenden Stoffe mit einer Mischung wie hierin beschrieben oder einer Riech- und/oder Aromastoffkomposition wie hierin beschrieben oder den Substanzen der Formel (Ia) 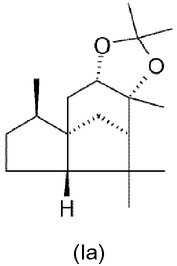 und der Formel (Ib), 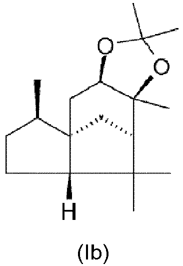 in einem Gewichtsverhältnis wie hierin definiert,
wobei die Menge an Mischung wie hierin beschrieben bzw. an Verbindung der Formel (Ia) und Verbindung der Formel (Ib) ausreicht, um (a) den bzw. die angenehmen Geruchseindrücke des bzw. der angenehm riechenden Stoffe zu verstärken und/oder (b) den bzw. die unangenehmen Geruchseindrücke des bzw. der unangenehm riechenden Stoffe zu vermindern oder zu maskieren.

Für die vorstehend beschriebenen erfindungsgemäßen Aspekte gilt vorzugsweise jeweils das oben im Zusammenhang mit einer erfindungsgemäßen Mischung, insbesondere einer bevorzugten Ausgestaltung davon, Gesagte entsprechend.

Die vorliegende Erfindung wird anhand der nachfolgenden Beispiele näher erläutert. Sofern nichts Anderes angegeben ist, beziehen sich dabei sämtliche Angaben auf das Gewicht.

### Beispiel 1: Herstellung einer Mischung im Wesentlichen bestehend aus Verbindung der Formel (la) und Verbindung der Formel (Ib)

In einem Rührgefäß werden 60 kg Dimethoxypropan (95 %ig) in 62 kg Aceton vorgelegt und mit 50 kg einer Isomerenmischung im Wesentlichen bestehend aus Verbindungen der Formel (III) (Cedrandiol), wobei die Isomerenmischung im Wesentlichen aus alpha,alpha-Cedrandiol der Formel (IIIa) und beta,beta-Cedrandiol der Formel (IIIb) besteht und wobei das Gewichtsverhältnis von der Verbindung der Formel (IIIa) zur Verbindung der Formel (IIIb) in der Isomerenmischung im Bereich von 95 : 5 bis 99,9 : 0,1 liegt, versetzt. Anschließend wird über einen Zeitraum von 2 Stunden eine Lösung bestehend aus 53 kg Aceton und 0,167 kg technischer Schwefelsäure bei einer Temperatur von höchstens 30 °C zugegeben. Nach einer weiteren Rührzeit von 4 Stunden wird die Reaktionsmischung mit einer Aufschlämmung bestehend aus 1,6 kg kalzinierter Soda in 5 kg Wasser auf einen pH-Wert von mindestens 8 eingestellt.

Bei anschließender Destillation werden aus der Reaktionsmischung die Leichtsieder soweit entfernt, dass eine Sumpftemperatur von 95 °C nicht überschritten wird. Nach beendeter Destillation wird der Destillationsrückstand mit 38 kg Methyl-tert.-Butylether versetzt und bei einer Temperatur von etwa 35 °C für ca. 30 Minuten gerührt. Anschließend lässt man die Reaktionsmischung ruhen, bis ein klares Zwei-Phasen-Gemisch entsteht. Die wässrige Phase wird abgetrennt, und die verbleibende organische Phase mit 12 kg Wasser versetzt. Die erhaltene Mischung wird bei einer Temperatur von etwa 35 °C für ca. 30 Minuten gerührt. Anschließend lässt man die Reaktionsmischung ruhen, bis ein klares Zwei-Phasen-Gemisch entsteht. Die wässrige Phase wird abgetrennt, und bei anschließender Destillation der organischen Phase wird Methyl-tert.-Butylether soweit entfernt, dass eine Sumpftemperatur von 95 °C bei 40 mbar nicht überschritten wird. Der Destillationsrückstand wird in 100 kg Heptan aufgenommen und mit einer wässrigen ethanolischen Lösung umkristallisiert.

Das erhaltene Reaktionsprodukt ist eine Mischung zu insgesamt 95 Gew.-% bestehend aus Verbindung der Formel (Ia) und Verbindung der Formel (Ib), wobei das Gewichtsverhältnis von der Verbindung der Formel (Ia) zur Verbindung der Formel (Ib) in der Mischung bei 95 : 5 liegt und 98 Gew.-% der Gesamtmenge an Verbindungen der Formeln (Ia) und (Ib) in der Mischung in kristalliner Form vorliegen.

Der Anteil an kristalliner Substanz wurde gaschromatographisch bestimmt.

### Beispiel 2: Parfümöle

### Reaktionsprodukt aus Beispiel 1 im Umfeld aldehydisch am Beispiel des folgenden Akkords:

| Bestandteile | FA | FB | FC | FD | FE | FF |
|---|---|---|---|---|---|---|
| Aldehyde C11 MOA 10% | 285,00 | 285,00 | 285,00 | 285,00 | 285,00 | 285,00 |
| 2-Methyldecanal | | | | | | |
| Aldehyde C11 10% | 95,00 | 95,00 | 95,00 | 95,00 | 95,00 | 95,00 |
| Undecanal | | | | | | |
| Aldehyde C12 MNA 10% | 38,00 | 38,00 | 38,00 | 38,00 | 38,00 | 38,00 |
| 2-Methylundecanal | | | | | | |
| Farenal® 10% | 76,00 | 76,00 | 76,00 | 76,00 | 76,00 | 76,00 |
| 2,6,10-Trimethyl-undec-9-enal | | | | | | |
| Florazon 10% | 209,00 | 209,00 | 209,00 | 209,00 | 209,00 | 209,00 |
| 3-(4-Ethylphenyl)-2,2-dimethylpropanal | | | | | | |
| Limonenal 10% | 95,00 | 95,00 | 95,00 | 95,00 | 95,00 | 95,00 |
| 3-(4-Methyl-1-cyclohex-3-enyl)-butanal | | | | | | |
| Mandarin Aldehyde 10% TEC | 114,00 | 114,00 | 114,00 | 114,00 | 114,00 | 114,00 |
| (E)-Dodec-2-enal | | | | | | |
| Ozonil 10% | 38,00 | 38,00 | 38,00 | 38,00 | 38,00 | 38,00 |
| Tridec-2-enenitril | | | | | | |
| Reaktionsprodukt aus Beispiel 1 | - | 0,50 | 1,00 | 5,00 | 10,00 | 30,00 |
| DPG | 50,00 | 49,50 | 49,00 | 45,00 | 40,00 | 20,00 |
| Summe | 1.000,00 | 1.000,00 | 1.000,00 | 1.000,00 | 1.000,00 | 1.000,00 |

In kleinen Dosierungen wie FB und FC ist eine deutlich stärkere und strahlendere Entwicklung des Duftes zu beobachten. In FC und FD wird zudem der typische aldehydige, fettige und metallische Charakter des Akkords hervorgehoben, was auf Grund des unterschiedlichen Duftprofils eher überraschend ist. Ab FE überwiegt der Einfluss vom Reaktionsprodukt aus Beispiel 1 und gibt dem Akkord einen stark ambrierten Geruch. Speziell im Vergleich zu Ambrocenide® 10DPG integriert sich das Reaktionsprodukt aus Beispiel 1 besser in den Akkord und unterstreicht seine Stärken, wie Sauberkeit und Frische, während Ambrocenide® 10DPG auch Stärke verleiht, aber durch seine holzigen Elemente nicht so klar und sauber wirkt.

### Reaktionsprodukt aus Beispiel 1 im Umfeld Citrus am Beispiel des folgenden Akkords:

| Bestandteile | AA | AB | AC | AE | AF | AG |
|---|---|---|---|---|---|---|
| Amarocit® | 60,00 | 60,00 | 60,00 | 60,00 | 60,00 | 60,00 |
| 6,6-Dimethoxy-2,5,5-trimethylhex-2-en | | | | | | |
| Lemon Oil Ital. | 210,00 | 210,00 | 210,00 | 210,00 | 210,00 | 210,00 |
| Orange Terpenes | 80,00 | 80,00 | 80,00 | 80,00 | 80,00 | 80,00 |
| Claritone® | 80,00 | 80,00 | 80,00 | 80,00 | 80,00 | 80,00 |
| 2,4,4,7-Tetramethyloct-6-en-3-on | | | | | | |
| Dihydromyrcenol | 150,00 | 150,00 | 150,00 | 150,00 | 150,00 | 150,00 |
| 2,6-Dimethyloct-7-en-2-ol | | | | | | |
| Linalool | 150,00 | 150,00 | 150,00 | 150,00 | 150,00 | 150,00 |
| 3,7-Dimethylocta-1,6-dien-3-ol | | | | | | |
| Linalylacetate | 50,00 | 50,00 | 50,00 | 50,00 | 50,00 | 50,00 |
| 3,7-Dimethylocta-1,6-dien-3-yl acetat | | | | | | |
| Terpinylacetate | 50,00 | 50,00 | 50,00 | 50,00 | 50,00 | 50,00 |
| (4-Methyl-1-propan-2-yl-1-cyclohex-2-enyl) acetat | | | | | | |
| Vertacetal® Coeur | 20,00 | 20,00 | 20,00 | 20,00 | 20,00 | 20,00 |
| 2,4,6-Trimethyl-4-phenyl-1,3-dioxan | | | | | | |
| Reaktionsprodukt aus Beispiel 1 | - | 10,00 | 30,00 | 50,00 | 100,00 | 150,00 |
| DPG | 150,00 | 140,00 | 120,00 | 100,00 | 150,00 | - |
| Summe | 1.000,00 | 1.000,00 | 1.000,00 | 1.000,00 | 1.100,00 | 1.000,00 |

Das Reaktionsprodukt aus Beispiel 1 fügt sich trotz seines ambrierten Duftes gut in die Note ein und unterstreicht den natürlichen citrus Charakter des Akkords mit citrustypischen lactonigen Untertönen. In höheren Dosierungen entwickelt es sich zu einem eher parfümistischen Akkord, in dem das Reaktionsprodukt aus Beispiel 1 die Fond Note darstellt. Speziell im Vergleich zu Ambrocenide® 10DPG integriert es sich besser in den Duft, wirkt klarer, strahlender und vor allem natürlicher.

### Reaktionsprodukt aus Beispiel 1 im Umfeld Veilchen am Beispiel des folgenden Akkords:

| Bestandteile | AA | AD | AF | AG | AH | AI |
|---|---|---|---|---|---|---|
| Cetone Alpha | | | | | | |
| (E)-3-Methyl-4-(2,6,6-trimethyl-1-cyclohex-2-enyl)but-3-en-2-on | 10,00 | 10,00 | 10,00 | 10,00 | 10,00 | 10,00 |
| Evernyl 10% | 20,00 | 20,00 | 20,00 | 20,00 | 20,00 | 20,00 |
| Methyl 2,4-dihydroxy - 3,6-dimethylbenzoat | | | | | | |
| Frambinon® 1% | 40,00 | 40,00 | 40,00 | 40,00 | 40,00 | 40,00 |
| 4-(4-Hydroxyphenyl) butan-2-on | | | | | | |
| Helional | 5,00 | 5,00 | 5,00 | 5,00 | 5,00 | 5,00 |
| 3-(1,3-Benzodioxol-5-yl)-2-methyl-propanal | | | | | | |
| Hedione | 60,00 | 60,00 | 60,00 | 60,00 | 60,00 | 60,00 |
| Methyl 3-oxo-2-pentyl-cyclopentanacetat | | | | | | |
| Heliotropin | 10,00 | 10,00 | 10,00 | 10,00 | 10,00 | 10,00 |
| 1,3-Benzodioxol-5-carbaldehyd | | | | | | |
| lonon Alpha | | | | | | |
| (E)-4-(2,6,6-Trimethyl-1-cyclohex-2-enyl)but-3-en-2-on | 80,00 | 80,00 | 80,00 | 80,00 | 80,00 | 80,00 |
| Ionon Beta | | | | | | |
| 4-(2,6,6-Trimethyl-cyclohexen-1-yl)but-3-en-2-on | 40,00 | 40,00 | 40,00 | 40,00 | 40,00 | 40,00 |
| Iraldein Beta | | | | | | |
| (E)-1-(2,6,6-trimethyl-cyclohexen-1-yl)pent-1-en-3-on | 110,00 | 110,00 | 110,00 | 110,00 | 110,00 | 110,00 |
| Iron Alpha 10% | | | | | | |
| (E)-4-[(1S)-1,2,6,6-tetramethylcyclohex-2-en-1-yl]but-3-en-2-on | 20,00 | 20,00 | 20,00 | 20,00 | 20,00 | 20,00 |
| Isoraldein 70 | | | | | | |
| 1-(2,6,6-Trimethyl-1-cyclohex-2-enyl)pent-1-en-3-on | 425,00 | 425,00 | 425,00 | 425,00 | 425,00 | 425,00 |
| Leafovert® 10% | 15,00 | 15,00 | 15,00 | 15,00 | 15,00 | 15,00 |
| [(Z)-Hex-3-enyl] methyl carbonat | | | | | | |
| Nonadienal 0.1% | | | | | | |
| (2E,6Z)-nona-2,6-dienal + (2E,4E)-nona-2,4-dienal | 10,00 | 10,00 | 10,00 | 10,00 | 10,00 | 10,00 |
| Parmanyl® | 20,00 | 20,00 | 20,00 | 20,00 | 20,00 | 20,00 |
| 3-[(Z)-Hex-3-enoxy]propannitril | | | | | | |
| Patchouli Oil | 5,00 | 5,00 | 5,00 | 5,00 | 5,00 | 5,00 |
| Syvertal 10% | 10,00 | 10,00 | 10,00 | 10,00 | 10,00 | 10,00 |
| 2-Heptan-3-yl-1,3-dioxolan | | | | | | |
| Violet Leaves Abs. 1% | 20,00 | 20,00 | 20,00 | 20,00 | 20,00 | 20,00 |
| Reaktionsprodukt aus Beispiel 1 | - | 5,00 | 10,00 | 20,00 | 50,00 | 100,00 |
| DPG | 100,00 | 95,00 | 90,00 | 80,00 | 50,00 | - |
| Summe | 1.000,00 | 1.000,00 | 1.000,00 | 1.000,00 | 1.000,00 | 1.000,00 |

Speziell in kleinen Dosierungen wie in AD wird durch das Reaktionsprodukt aus Beispiel 1 ein Iriswurzel Aspekt deutlich hervorgehoben. In AF unterstreicht es die Ionon-Elemente des Akkords. In AG wird der ambrierte Charakter deutlich erkennbar, jedoch nicht unangenehm.

### Reaktionsprodukt aus Beispiel 1 im Umfeld Moschus am Beispiel des folgenden Akkords:

| Bestandteile | AA | AB | AC | AD | AE | AF |
|---|---|---|---|---|---|---|
| Aurelione | 100,00 | 100,00 | 100,00 | 100,00 | 100,00 | 100,00 |
| (7E)-cyclohexadec-7-en-1-on | | | | | | |
| Globalide | 300,00 | 300,00 | 300,00 | 300,00 | 300,00 | 300,00 |
| (3E)-Oxacyclo-hexadec-3-en-2-on | | | | | | |
| Globanone | 200,00 | 200,00 | 200,00 | 200,00 | 200,00 | 200,00 |
| (8E)-Cyclohexadec-8-en-1-on | | | | | | |
| Macrolide Supra | 200,00 | 200,00 | 200,00 | 200,00 | 200,00 | 200,00 |
| 16-Oxacyclohexa-decan-1-on | | | | | | |
| Galaxolid | | | | | | |
| 1,3,4,6,7,8-Hexahydro-4,6,6,7,8,8-hexamethyl-cyclopenta(g)-2-benzopyran | 100,00 | 100,00 | 100,00 | 100,00 | 100,00 | 100,00 |
| Reaktionsprodukt aus Beispiel 1 | - | 1,00 | 5,00 | 90,00 | 50,00 | 100,00 |
| DPG | 100,00 | 99,00 | 95,00 | 10,00 | 50,00 | - |
| Summe | 1.000,00 | 1.000,00 | 1.000,00 | 1.000,00 | 1.000,00 | 1.000,00 |

Das Reaktionsprodukt aus Beispiel 1 unterstützt überraschend gut den weichen Moschus Charakter des Akkords. Es verleiht dem als Fond Note bekannten Moschus eine bessere Wahrnehmbarkeit und Stärke bereits im Angeruch. In höheren Dosierungen ab AD wird die Amber Note deutlich erkennbar, wird aber auch bis AF nicht als negativ empfunden sondern bildet einen schönen harmonischen Komplex. Im Vergleich dazu zeigt sich Ambrocenide® 10DPG deutlich flacher und weniger strahlend.

### Reaktionsprodukt aus Beispiel 1 im Umfeld holzig am Beispiel des folgenden Akkords:

| Bestandteile | AA | AB | AC | AD | AE |
|---|---|---|---|---|---|
| Iso E Super | 320,00 | 320,00 | 320,00 | 320,00 | 320,00 |
| 1-(2,3,8,8-Tetramethyl-1,3,4,5,6,7-hexa-hydronaphthalin-2-yl)ethanon | | | | | |
| Ysamber® K | 230,00 | 230,00 | 230,00 | 230,00 | 230,00 |
| spiro[1,3-dioxolane-2,5'-(4',4',8',8'-tetramethyl-hexahydro-3',9'-methanonaphthalin)] | | | | | |
| Cedramber | 30,00 | 30,00 | 30,00 | 30,00 | 30,00 |
| [3R-(3α,3aβ,7β,8aα)]-octahydro-6-methoxy-3,6,8,8-tetramethyl-1H-3a,7-methanoazulen | | | | | |
| Vertofix | 70,00 | 70,00 | 70,00 | 70,00 | 70,00 |
| [3R-(3α,3aβ,7β,8aα)]-1-(2,3,4,7,8,8a-hexahydro-3,6,8,8-tetramethyl-1H-3a,7-methanoazulen-5-yl)ethan-1-on | | | | | |
| Timberol® | 200,00 | 200,00 | 200,00 | 200,00 | 200,00 |
| 1-(2,2,6-Trimethyl-cyclohexyl)hexan-3-ol | | | | | |
| Reaktionsprodukt aus Beispiel 1 | - | 20,00 | 50,00 | 100,00 | 150,00 |
| DPG | 150,00 | 130,00 | 100,00 | 150,00 | - |
| Summe | 1.000,00 | 1.000,00 | 1.000,00 | 1.100,00 | 1.000,00 |

Das Reaktionsprodukt aus Beispiel 1 unterstützt in AB den holzigen Charakter des Akkords und verleiht ihm Strahlung und Stärke. In AC macht es den Akkord trocken holziger und gibt ihm einen ambrierten Unterton. In den höheren Dosierungen bildet sich eine Holz-Amber Akkord.

### Reaktionsprodukt aus Beispiel 1 im Umfeld Amber am Beispiel des folgenden Akkords:

| Bestandteile | CA | CB | CC | CD | CE | CF |
|---|---|---|---|---|---|---|
| Amberwood® F | 200,00 | 200,00 | 200,00 | 200,00 | 200,00 | 200,00 |
| Ethoxymethoxycyclododecan | | | | | | |
| Cashmeran | 40,00 | 40,00 | 40,00 | 40,00 | 40,00 | 40,00 |
| 1,1,2,3,3-Pentamethyl-2,5,6,7-tetrahydroinden-4-on | | | | | | |
| Iso E Super | 250,00 | 250,00 | 250,00 | 250,00 | 250,00 | 250,00 |
| 1-(2,3,8,8-Tetramethyl-1,3,4,5,6,7-hexa-hydronaphthalin-2-yl)ethanon | | | | | | |
| Madranol | 200,00 | 200,00 | 200,00 | 200,00 | 200,00 | 200,00 |
| 3-Methyl-4-(2,2,6-trimethylcyclohexyl)butan-2-ol | | | | | | |
| Ambrinol S 10% | 20,00 | 20,00 | 20,00 | 20,00 | 20,00 | 20,00 |
| 2,5,5-Trimethyl-1,3,4,4a,6,7-hexahydronaphthalin-2-ol | | | | | | |
| Ambroxide Cryst. | 110,00 | 110,00 | 110,00 | 110,00 | 110,00 | 110,00 |
| (3aR,5aS,9aS,9bR)-3a,6,6,9a-Tetramethyl-2,4,5,5a,7,8,9,9b-octahydro-1H-benzo[e][1]benzofuran | | | | | | |
| Ysamber® K | 100,00 | 100,00 | 100,00 | 100,00 | 100,00 | 100,00 |
| spiro[1,3-dioxolane-2,5'-(4',4',8',8'-tetram-ethyl-hexahydro-3',9'-methanonaphthalin)] | | | | | | |
| Ambrarome 10% | 25,00 | 25,00 | 25,00 | 25,00 | 25,00 | 25,00 |
| Base aus verschiedenen Bestandteilen | | | | | | |
| Lactoscatone | 5,00 | 5,00 | 5,00 | 5,00 | 5,00 | 5,00 |
| Hexahydro-3,5,5-trimethyl-3,8a-ethano-8ah-1-benzopyran-2(3H)-on | | | | | | |
| Reaktionsprodukt aus Beispiel 1 | - | 1,00 | 5,00 | 10,00 | 20,00 | 50,00 |
| DPG | 50,00 | 49,00 | 45,00 | 40,00 | 30,00 | - |
| Summe | 1.000,00 | 1.000,00 | 1.000,00 | 1.000,00 | 1.000,00 | 1.000,00 |

Das Reaktionsprodukt aus Beispiel 1 unterstützt von der niedrigsten bis zu höchsten Dosierung die Amber Note, verleiht ihr Komplexität, macht die Note strahlender, ambrierter und lebendiger. Im Vergleich dazu verstärkt Ambrocenide® 10DPG den Akkord auch, bleibt aber ansonsten eher im Hintergrund, es gibt Volumen, dreht aber in den höheren Dosierungen eher in eine etwas schmutzige, animalische Richtung ab.

### Beispiel 3: Parfümierte Produkte

### Waschpulver:

| **Bestandteil** | **Gew.-Anteile** |
|---|---|
| | |
| Aldehyde C11 Undecylic 10% | 16,00 |
| Aldehyde C11 Undecylenic 10% | 18,00 |
| Aldehyde C12 Lauric 10% | 14,00 |
| Aldehyde C12 MNA 10% | 12,00 |
| Hexenal Trans-2 10% | 4,00 |
| Hexenyl Acetate Cis-3 | 4,00 |
| Vertocitral | 10,00 |
| Magnolan | 130,00 |
| Mintonat | 35,00 |
| Dihydro Myrcenol | 70,00 |
| Orange Oil | 35,00 |
| Nerolione 10% | 3,50 |
| Cantryl® | 3,50 |
| Hexyl Acetate | 18,00 |
| Jasmaprunat | 18,00 |
| Aldehyde C14 So-Called | 50,00 |
| Ethyl Methyl Butyrate-2 | 8,00 |
| Manzanate | 1,20 |
| Allyl Cyclohexyl Propionate | 8,00 |
| Aprifloren® | 3,00 |
| Fruitate | 1,80 |
| Ethyl Linalool | 56,00 |
| Dimethyl Benzyl Carbinyl Butyrate | 7,00 |
| Rose Abs. Type Base | 30,00 |
| Rosaphen® | 30,00 |
| Damascenone Total | 1,20 |
| Damascone Alpha | 1,80 |
| Benzyl Acetate | 28,00 |
| Hedione | 56,00 |
| Hexyl Cinnamic Aldehyde Alpha | 130,00 |
| Parmanyl® | 3,50 |
| Isoraldeine 70 | 28,00 |
| Isoeugenyl Acetate | 3,50 |
| Agrumex HC | 50,00 |
| Ambroxide Cryst. | 1,50 |
| Reaktionsprodukt aus Beispiel 1 | 0,80 |
| Dipropylene Glycol | 109,70 |
| Summe | 1.000,00 |

Das Reaktionsprodukt aus Beispiel 1 unterstreicht hier die warme holzige Fondnote und unterstützt die Haftung auf der Wäsche.

### Shampoo:

| **Bestandteil** | **Gew.-Anteile** |
|---|---|
| | |
| Hexenol Cis-3 | 2,50 |
| Galbanum Oil 10% | 5,00 |
| Magnolan | 25,00 |
| Bergamot Oil RCO | 80,00 |
| Linalyl Acetate | 120,00 |
| Lemon Oil | 80,00 |
| Neroli Base | 10,00 |
| Lavender Oil | 6,00 |
| Thyme Oil 10% | 6,00 |
| Linalool | 60,00 |
| Phenylethyl Alcohol BA Free | 20,00 |
| Vitessence® Rose De Mai | 6,50 |
| Narcisse Abs. 10% | 1,30 |
| Hedione | 80,00 |
| Jasmolactone Cis 10% | 6,00 |
| Parmanyl® 10% | 12,70 |
| Ionone Beta | 6,00 |
| Methyl lonone Gamma Pure | 18,00 |
| Irone Alpha 10% | 12,00 |
| Benzoin Siam Resin 50% | 5,00 |
| Coumarin | 5,00 |
| Iso E Super | 180,00 |
| Cashmeran | 12,00 |
| Isobutyl Quinoline 10% | 6,00 |
| Reaktionsprodukt aus Beispiel 1 | 0,2 |
| Globalide® | 45,00 |
| Dipropylene Glycol | 189,80 |
| Summe | 1.000,00 |

Das Reaktionsprodukt aus Beispiel 1 verstärkt hier sowohl die Kopfnote als auch die haftenden holzigen Elemente in der Fondnote.

## Patentansprüche

1. Mischung, enthaltend oder zumindest im Wesentlichen bestehend aus
- Verbindung der Formel (Ia) 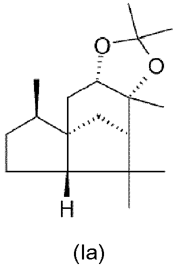 und
- Verbindung der Formel (Ib), 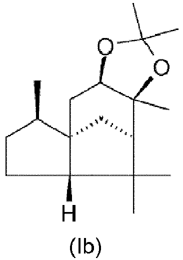
wobei das Gewichtsverhältnis von der Verbindung der Formel (Ia) zur Verbindung der Formel (Ib) in der Mischung im Bereich von 90 : 10 bis 99 : 1 liegt.

2. Mischung nach Anspruch 1, wobei das Gewichtsverhältnis von der Verbindung der Formel (Ia) zur Verbindung der Formel (Ib) in der Mischung im Bereich von 95 : 5 bis 99 : 1, vorzugsweise bei etwa 98 : 2, liegt,
und/oder wobei die Mischung zudem eine Verbindung der Formel (II) (Cedralon) enthält, 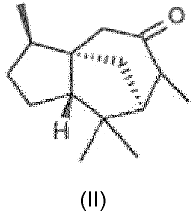 wobei das Gewichtsverhältnis der Gesamtmenge an Verbindungen der Formeln (Ia) und (Ib) zur Gesamtmenge an Verbindung der Formel (II) in der Mischung wenigstens 6 : 1, vorzugsweise wenigstens 9 : 1, weiter bevorzugt wenigstens 12 : 1, besonders bevorzugt wenigstens 25 : 1, ist.

3. Mischung nach einem der Ansprüche 1 oder 2, wobei die Mischung zudem eine Verbindung der Formel (III) (Cedrandiol) enthält, 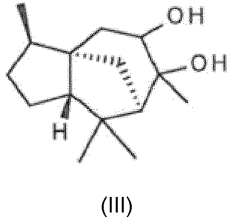 wobei das Gewichtsverhältnis der Gesamtmenge an Verbindungen der Formeln (Ia) und (Ib) zur Gesamtmenge an Verbindung der Formel (III) in der Mischung wenigstens 15 : 1, vorzugsweise wenigstens 20 : 1, besonders bevorzugt wenigstens 25 : 1, ist.

4. Mischung nach einem der Ansprüche 1 bis 3, wobei wenigstens 90 Gew.-% der Gesamtmenge an Verbindungen der Formeln (Ia) und (Ib) in der Mischung in kristalliner Form vorliegen.

5. Mischung nach einem der Ansprüche 1 bis 4, herstellbar durch ein Verfahren umfassend die nachfolgenden Schritte:
a) Bereitstellen einer Isomerenmischung, enthaltend oder zumindest im Wesentlichen bestehend aus Verbindungen der Formel (III) (Cedrandiol), wobei die Isomerenmischung alpha,alpha-Cedrandiol der Formel (IIIa) 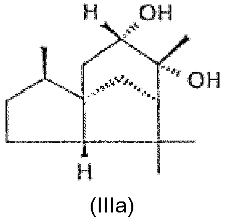 und beta,beta-Cedrandiol der Formel (IIIb) 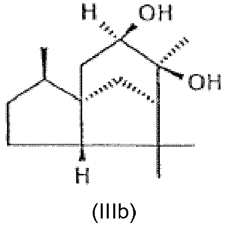 enthält oder zumindest im Wesentlichen daraus besteht, und wobei das Gewichtsverhältnis von der Verbindung der Formel (IIIa) zur Verbindung der Formel (IIIb) in der Isomerenmischung im Bereich von 95 : 5 bis 99,9 : 0,1 liegt, vorzugsweise wenigstens 98 : 2 beträgt,
b) Umsetzen der Isomerenmischung aus Schritt a) mit Dimethoxypropan im Molverhältnis von wenigstens 1 : 2, bezogen auf die Gesamt-Molmenge an Verbindungen der Formel (III) zur Gesamt-Molmenge an Dimethoxypropan, wobei das Verfahren vorzugsweise zudem folgenden Schritt umfasst:
c) Auskristallisieren des Reaktionsprodukts aus Schritt b) aus wässriger alkoholischer Lösung.

6. Verfahren zur Herstellung einer Mischung, enthaltend oder zumindest im Wesentlichen bestehend aus
- Verbindung der Formel (Ia) 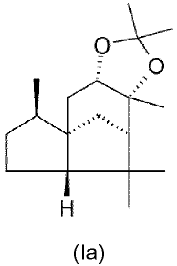 und
- Verbindung der Formel (Ib), 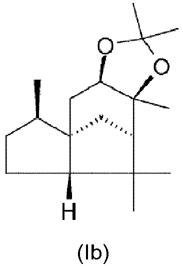
wobei das Gewichtsverhältnis von der Verbindung der Formel (Ia) zur Verbindung der Formel (Ib) in der Mischung im Bereich von 90 : 10 bis 99 : 1 liegt,
vorzugsweise zur Herstellung einer Mischung nach einem der Ansprüche 1 bis 5, umfassend die folgenden Schritte:
a) Bereitstellen einer Isomerenmischung, enthaltend oder zumindest im Wesentlichen bestehend aus Verbindungen der Formel (III) (Cedrandiol), wobei die Isomerenmischung alpha,alpha-Cedrandiol der Formel (IIIa) 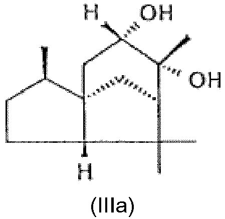 und beta,beta-Cedrandiol der Formel (IIIb) 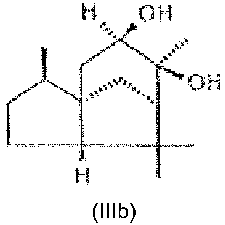 enthält oder zumindest im Wesentlichen daraus besteht und wobei das Gewichtsverhältnis von der Verbindung der Formel (IIIa) zur Verbindung der Formel (IIIb) in der Isomerenmischung im Bereich von 95 : 5 bis 99,9 : 0,1 liegt, vorzugsweise wenigstens 98 : 2 beträgt,
b) Umsetzen der Isomerenmischung aus Schritt a) mit Dimethoxypropan im Molverhältnis von wenigstens 1 : 2, bezogen auf die Gesamt-Molmenge an Verbindungen der Formel (III) zur Gesamt-Molmenge an Dimethoxypropan, vorzugsweise umfassend den zusätzlichen Schritt
c) Auskristallisieren des Reaktionsprodukts aus Schritt b) aus wässriger alkoholischer Lösung.

7. Riech- und/oder Aromastoffkomposition, vorzugsweise Parfümöl, enthaltend oder bestehend aus einer Mischung nach einem der Ansprüche 1 bis 5 und zudem vorzugsweise einem oder mehreren zusätzlichen Riech- und/oder Aromastoffen,
vorzugsweise wobei der zusätzliche bzw. einer, mehrere oder sämtliche der zusätzlichen Riechstoffe ausgewählt ist bzw. sind aus der Gruppe bestehend aus 3-(4-Methyl-1-cyclohex-3-enyl)-butanal, 4-(4-Hydroxyphenyl) butan-2-on, (E)-4-(2,6,6-Trimethyl-1-cyclohex-2-enyl)but-3-en-2-on, (E)-4-(2,6,6-Trimethyl-1-cyclohex-2-enyl)but-3-en-2-on, (E)-1-(2,6,6-trimethyl-cyclohexen-1-yl)pent-1-en-3-on, (E)-4-[(1S)-1,2,6,6-tetramethylcyclohex-2-en-1-yl]-but-3-en-2-on, 1-(2,6,6-Trimethyl-1-cyclohex-2-enyl)pent-1-en-3-on, [(Z)-Hex-3-enyl] methyl carbonat, 3-[(Z)-Hex-3-enoxy]propannitril, 1-(2,3,8,8-Tetramethyl-1,3,4,5,6,7-hexahydronaphthalin-2-yl)ethanon, spiro[1,3-dioxolan-2,5'-(4',4',8',8'-tetramethyl-hexahydro-3',9'-methanonaphthalin)], [3R-(3α,3aβ,6β,7β,8aα)]-octa-hydro-6-methoxy-3,6,8,8-tetramethyl-1H-3a,7-methanoazulen,[3R-(3α,3aβ,7β,8aα)] -1-(2,3,4,7,8,8a-hexahydro-3,6,8,8-tetramethyl-1H-3a,7-methano-azulen-5-yl) ethan-1-on, 1-(2,2,6-Trimethyl-cyclohexyl) hexan-3-ol, 6,6-Dimethoxy-2,5,5-trimethylhex-2-en, 2,6-Dimethyloct-7-en-2-ol, 3,7-Dimethylocta-1,6-dien-3-ol, (3,7-Dimethylocta-1,6-dien-3-yl)acetat, (4-Methyl-1-propan-2-yl-1-cyclohex-2-enyl) acetat, (8E)-Cyclohexadec-8-en-1-on, 16-Oxacyclohexa-decan-1-on, 1,3,4,6,7,8-Hexahydro-4,6,6,7,8,8-hexamethyl-cyclopenta(g)-2-benzopyran, Ethoxymethoxy-cyclododecan, 1,1,2,3,3-Pentamethyl-2,5,6,7-tetrahydroinden-4-on, 1-(2,3,8,8-Tetramethyl-1,3,4,5,6,7-hexa-hydronaphthalin-2-yl)ethanon.

8. Riech- und/oder Aromastoffkomposition nach Anspruch 7, wobei die Menge an Mischung nach einem der Ansprüche 1 bis 5 bzw. die Menge an Verbindung der Formel (Ia) und Verbindung der Formel (Ib) ausreicht,
(a) um den bzw. einen oder mehrere unangenehme Geruchseindrücke eines anderen Riech- und/oder Aromastoffs in der Riech- und/oder Aromastoffkomposition zu maskieren oder zu vermindern,
und/oder
(b) um den bzw. einen oder mehrere angenehme Geruchseindrücke eines anderen Riech- und/oder Aromastoffs in der Riech- und/oder Aromastoffkomposition zu verstärken.

9. Riech- und/oder Aromastoffkomposition nach einem der Ansprüche 7 oder 8, vorzugsweise Parfümöl, wobei die Gesamtmenge an Verbindung der Formel (Ia) und Verbindung der Formel (Ib) bezogen auf das Gesamtgewicht der Riech- und/oder Aromastoffkomposition 0,01 bis 10 Gew.-%, vorzugsweise 0,03 bis 5 Gew.-%, besonders bevorzugt 0,05 bis 2 Gew.-%, beträgt.

10. Parfümiertes Produkt, enthaltend eine Mischung nach einem der Ansprüche 1 bis 5 oder, vorzugsweise, eine Riech- und/oder Aromastoffkomposition nach einem der Ansprüche 7 bis 9, vorzugsweise ein Parfümöl, in einer sensorisch wirksamen Menge, wobei der Anteil der Mischung bzw. der Riech- und/oder Aromastoffkomposition bezogen auf das Gesamtgewicht des Produkts vorzugsweise im Bereich von 0,01 bis 10 Gew.-%, bevorzugt im Bereich von 0,1 bis 5 Gew.-%, besonders bevorzugt im Bereich von 0,25 bis 3 Gew.-%, liegt,
vorzugsweise wobei das Produkt ausgewählt ist aus der Gruppe bestehend aus Parfüm-Extraits, Eau de Parfums, Eau de Toilettes, Ra-sierwässer, Eau de Colognes, Pre-shave-Produkte, Splash-Colognes, parfümierte Erfrischungstücher, saure, alkalische und neutrale Reinigungsmittel, Textilerfrischer, Bügelhilfen, flüssige Waschmittel, pulverförmige Waschmittel, Wä-schevorbehandlungsmittel, Wäscheweichspüler, Waschseifen, Waschtabletten, Desinfektionsmittel, Oberflächendesinfektionsmittel, Luftverbesserer, Aerosolsprays, Wachse und Polituren, Körperpflegemittel, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien und Antiperspirantien, Produkte der dekorativen Kosmetik, Kerzen, Lampenöle, Räucherstäbchen, Insektizide, Repellentien und Treibstoffe.

11. Verfahren zum Herstellen eines parfümierten Produkts, insbesondere eines parfümierten Produkts nach Anspruch 10, umfassend folgende Schritte:
i) Bereitstellen einer Mischung nach einem der Ansprüche 1 bis 5 oder einer Riech- und/oder Aromastoffkomposition nach einem der Ansprüche 7 bis 9 oder der Substanzen der Formel (Ia) 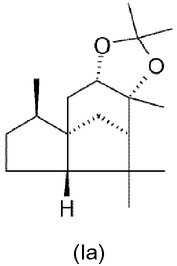 und der Formel (Ib), 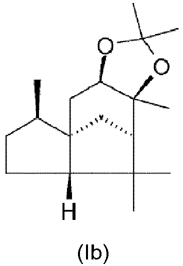 in einem Gewichtsverhältnis wie in Anspruch 1 oder 2 definiert,
ii) Bereitstellen eines oder mehrerer weiterer Bestandteile des herzustellenden parfümierten Produkts, und
iii) Inkontaktbringen oder Mischen der in Schritt ii) bereitgestellten weiteren Bestandteile mit einer sensorisch wirksamen Menge der in Schritt i) bereitgestellten Bestandteile.

12. Verwendung einer Mischung nach einem der Ansprüche 1 bis 5
(a) zum Maskieren oder Vermindern des bzw. eines oder mehrerer unangenehmer Geruchseindrücke eines oder mehrerer unangenehm riechender Stoffe,
und/oder
(b) zum Verstärken des bzw. eines oder mehrerer angenehmer Geruchseindrücke eines oder mehrerer angenehm riechender Stoffe.

13. Verwendung nach Anspruch 12 in einer Zusammensetzung, vorzugsweise einem Parfümöl, die bzw. das einen oder mehrere (weitere) angenehm und/oder unangenehm riechende Stoffe enthält, deren unangenehmer Geruchseindruck durch die Mischung nach einem der Ansprüche 1 bis 5 maskiert oder vermindert und/oder deren angenehmer Geruchseindruck durch die Mischung nach einem der Ansprüche 1 bis 5 verstärkt wird, wobei diese(r) angenehm und/oder unangenehm riechende Stoff bzw. einer, mehrere oder sämtliche dieser angenehm und/oder unangenehm riechende Stoffe ausgewählt ist bzw. sind aus der Gruppe bestehend aus 3-(4-Methyl-1-cyclohex-3-enyl)-butanal, 4-(4-Hydroxyphenyl) butan-2-on, (E)-4-(2,6,6-Trimethyl-1-cyclohex-2-enyl)but-3-en-2-on, (E)-4-(2,6,6-Trimethyl-1-cyclohex-2-enyl)but-3-en-2-on, (E)-1-(2,6,6-trimethyl-cyclohexen-1-yl)pent-1-en-3-on, (E)-4-[(1S)-1,2,6,6-tetramethylcyclohex-2-en-1-yl]-but-3-en-2-on, 1-(2,6,6-Trimethyl-1-cyclohex-2-enyl)pent-1-en-3-on, [(Z)-Hex-3-enyl] methyl carbonat, 3-[(Z)-Hex-3-enoxy]propannitril, 1-(2,3,8,8-Tetramethyl-1,3,4,5,6,7-hexa-hydro-naphthalin-2-yl)ethanon, spiro[1,3-dioxolan-2,5'-(4',4',8',8'-tetramethyl-hexahydro-3',9'-methanonaphthalin)], [3R-(3α,3aβ,6β,7β,8aα)]-octa-hydro-6-methoxy-3,6,8,8-tetramethyl-1H-3a,7-methanoazulen,[3R-(3α,3aβ,7β,8aα)]-1-(2,3,4,7,8,8a-hexa-hydro-3,6,8,8-tetramethyl-1H-3a,7-methano-azulen-5-yl)ethan-1-on, 1-(2,2,6-Trimethyl-cyclohexyl)hexan-3-ol, 6,6-Dimethoxy-2,5,5-trimethylhex-2-en, 2,6-Dimethyloct-7-en-2-ol, 3,7-Dimethylocta-1,6-dien-3-ol, (3,7-Dimethylocta-1,6-dien-3-yl)acetat, (4-Methyl-1-propan-2-yl-1-cyclohex-2-enyl) acetat, (8E)-Cyclohexadec-8-en-1-on, 16-Oxacyclohexa-decan-1-on, 1,3,4,6,7,8-Hexahydro-4,6,6,7,8,8-hexamethyl-cyclopenta(g)-2-benzopyran, Ethoxymethoxy-cyclododecan, 1,1,2,3,3-Pentamethyl-2,5,6,7-tetrahydroinden-4-on, 1-(2,3,8,8-Tetramethyl-1,3,4,5,6,7-hexa-hydronaphthalin-2-yl)ethanon.

14. Verwendung nach Anspruch 12 oder 13, zum Verstärken des bzw. eines oder mehrerer Geruchseindrücke ausgewählt aus der Gruppe bestehend aus den Geruchsnoten blumig, Amber, holzig, Moschus, Veilchen, Citrus und aldehydig.

15. Verfahren
(a) zum Maskieren oder Vermindern des bzw. eines oder mehrerer unangenehmer Geruchseindrücke eines oder mehrerer unangenehm riechender Stoffe,
und/oder
(b) zum Verstärken des bzw. eines oder mehrerer angenehmer Geruchseindrücke eines oder mehrerer angenehm riechender Stoffe,
umfassend folgenden Schritt:
Vermischen des bzw. der (a) angenehm und/oder (b) unangenehm riechenden Stoffe mit einer Mischung nach einem der Ansprüche 1 bis 5 oder einer Riech- und/oder Aromastoffkomposition nach einem der Ansprüche 7 bis 9 oder den Substanzen der Formel (Ia) 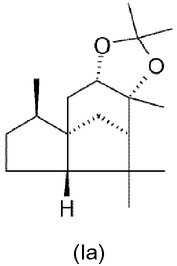 und der Formel (Ib), 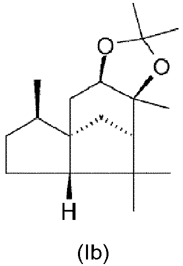 in einem Gewichtsverhältnis wie in Anspruch 1 oder 2 definiert,
wobei die Menge an Mischung nach einem der Ansprüche 1 bis 5 bzw. an Verbindung der Formel (Ia) und Verbindung der Formel (Ib) ausreicht, um (a) den bzw. die angenehmen Geruchseindrücke des bzw. der angenehm riechenden Stoffe zu verstärken und/oder (b) den bzw. die unangenehmen Geruchseindrücke des bzw. der unangenehm riechenden Stoffe zu vermindern oder zu maskieren.

## Claims

1. Mixture, comprising or at least substantially consisting of
- compound of formula (Ia) 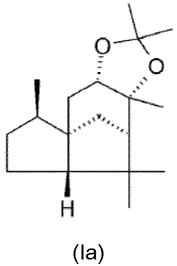 and
- compound of formula (Ib), 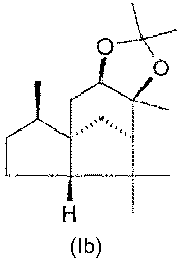
wherein the weight ratio of the compound of formula (Ia) to the compound of formula (Ib) in the mixture is in a range of from 90 : 10 to 99 : 1.

2. Mixture according to claim 1, wherein the weight ratio of the compound of formula (Ia) to the compound of formula (Ib) in the mixture is in a range of from 95 : 5 to 99 : 1, preferably about 98 : 2,
and/or wherein the mixture additionally comprises a compound of formula (II) (Cedralon), 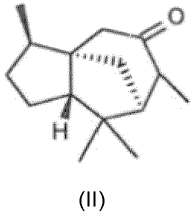 wherein the weight ratio of the total amount of the compounds of formulas (Ia) and (Ib) to the total amount of the compound of formula (II) in the mixture is at least 6 : 1, preferably at least 9 : 1, further preferably at least 12 : 1, particularly preferably at least 25 : 1.

3. Mixture according to one of the claims 1 or 2, wherein the mixture additionally comprises a compound of formula (III) (Cedrandiol), 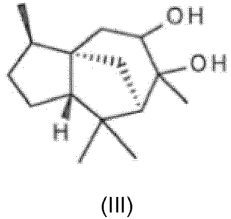 wherein the weight ratio of the total amount of the compounds of formulas (Ia) and (Ib) to the total amount of the compound of formula (III) in the mixture is at least 15 : 1, preferably at least 20 : 1, further preferably at least 25 : 1.

4. Mixture according to one of the claims 1 to 3, wherein at least 90 wt.-% of the total amount of the compounds of formulas (Ia) and (Ib) in the mixture are present in crystalline form.

5. Mixture according to one of the claims 1 to 4, preparable by a method comprising the following steps:
a) Providing a mixture of isomers, comprising or at least substantially consisting of compounds of formula (III) (Cedrandiol), wherein the mixture of isomers comprises or at least substantially consists of alpha,alpha-Cedrandiol of formula (IIIa) 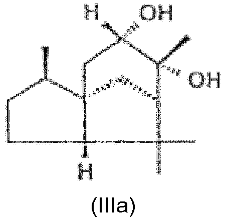 and beta,beta-Cedrandiol of formula (IIIb) 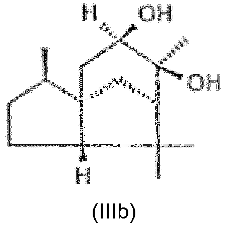 and wherein the weight ratio of the compound of formula (IIIa) to the compound of formula (IIIb) in the mixture of isomers is in a range of 95 : 5 to 99.9 : 0.1, preferably is at least 98 : 2,
b) reacting the mixture of isomers of step a) with Dimethoxypropane in a molar ratio of at least 1 : 2, related to the total amount of substance of the compounds of formula (III) to the total amount of substance of Dimethoxypropane,
wherein the method preferably and additionally comprises the following step:
c) crystallizing the reaction product of step b) from aqueous alcoholic solution.

6. Method for producing a mixture comprising or at least substantially consisting of
- compound of formula (Ia) 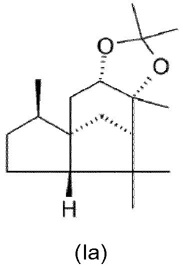 and
- compound of formula (Ib), 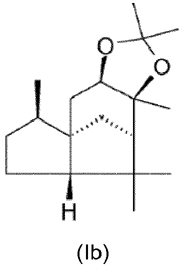
wherein the weight ratio of the compound of formula (Ia) to the compound of formula (Ib) in the mixture is in a range of from 90 : 10 to 99 : 1,
preferably for producing a mixture according to one of the claims 1 to 5,
comprising the following steps:
a) Providing a mixture of isomers, comprising or at least substantially consisting of compounds of formula (III) (Cedrandiol), wherein the mixture of isomers comprises or at least substantially consists of alpha,alpha-Cedrandiol of formula (IIIa) 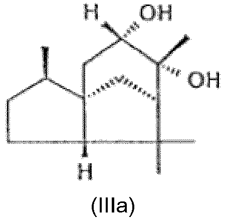 and beta,beta-Cedrandiol of formula (IIIb) 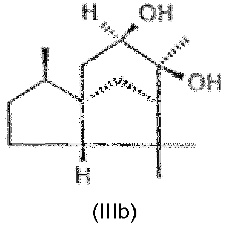 and wherein the weight ratio of the compound of formula (IIIa) to the compound of formula (IIIb) in the mixture of isomers is in a range of 95 : 5 to 99.9 : 0.1, preferably is at least 98 : 2,
b) reacting the mixture of isomers of step a) with Dimethoxypropane in a molar ratio of at least 1 : 2, related to the total amount of substance of the compounds of formula (III) to the total amount of substance of Dimethoxypropane,
preferably and additionally comprising the following additional step:
c) crystallizing the reaction product of step b) from aqueous alcoholic solution.

7. Flavouring composition and/or aromatic substance composition, preferably perfume oil, comprising or consisting of a mixture according to one of the claims 1 to 5 and preferably one or more additional flavourings and/or aromatic substances, preferably wherein the additional or one, more or all of the additional flavourings is/are selected from the group consisting of 3-(4-Methyl-1-cyclohex-3-enyl)-butanal, 4-(4-Hydroxyphenyl) butan-2-one, (E)-4-(2,6,6-Trimethyl-1-cyclohex-2-enyl)but-3-ene-2-one, (E)-4-(2,6,6-Trimethyl-1-cyclohex-2-enyl)but-3-ene-2-one, (E)-1-(2,6,6-trimethyl-cyclohexene-1-yl)pent-1-ene-3-one, (E)-4-[(1S)-1,2,6,6-tetramethylcyclohex-2-ene-1-yl]-but-3-ene-2-one, 1-(2,6,6-Trimethyl-1-cyclohex-2-enyl)pent-1-ene-3-one, [(Z)-Hex-3-enyl] methyl carbonate, 3-[(Z)-Hex-3-enoxy]propannitrile, 1-(2,3,8,8-Tetramethyl-1,3,4,5,6,7-hexa-hydronaphthalene-2-yl)ethanone, spiro[1,3-dioxolan-2,5'-(4',4',8',8'-tetramethyl-hexahydro-3',9'-methanonaphthalene)], [3R-(3α,3aβ,6β,7β,8aα)]-octa-hydro-6-methoxy-3,6,8,8-tetramethyl-1H-3a,7-methanoazulene, [3R-(3α,3aβ,7β,8aα)]-1-(2,3,4,7,8,8a-hexahydro-3,6,8,8-tetramethyl-1H-3a,7-methano-azulene-5-yl) ethane-1-one, 1-(2,2,6-Trimethyl-cyclohexyl) hexane-3-ol, 6,6-Dimethoxy-2,5,5-trimethylhex-2-ene, 2,6-Dimethyloct-7-ene-2-ol, 3,7-Dimethylocta-1,6-diene-3-ol, (3,7-Dimethylocta-1,6-diene-3-yl)acetate, (4-Methyl-1-propane-2-yl-1-cyclohex-2-enyl) acetate, (8E)-Cyclohexadec-8-ene-1-one, 16-Oxacyclohexa-decane-1-one, 1,3,4,6,7,8-Hexahydro-4,6,6,7,8,8-hexamethyl-cyclopenta(g)-2-benzopyrane, Ethoxymethoxy-cyclododecane, 1,1,2,3,3-Pentamethyl-2,5,6,7-tetrahydroinden-4-one, 1-(2,3,8,8-Tetramethyl-1,3,4,5,6,7-hexa-hydro-naphthalene-2-yl)ethanone.

8. Flavouring composition and/or aromatic substance composition according to claim 7, wherein the amount of mixture according to one of the claims 1 to 5 or the amount of the compound of formula (Ia) and compound of formula (Ib) is sufficient
(a) to mask or reduce the, one or more unpleasant olfactory impression(s) of another flavour and/or aromatic substance in the flavouring composition and/or aromatic substance composition,
and/or
(b) to enhance the, one or more pleasant olfactory impression(s) of another flavour and/or aromatic substance in the flavouring composition and/or aromatic substance composition.

9. Flavouring composition and/or aromatic substance composition according to one of the claims 7 or 8, preferably perfume oil, wherein the total amount of the compound of formula (Ia) and the compound of formula (Ib) related to the total weight of the flavouring composition and/or aromatic substance composition is 0.01 to 10 wt.-%, preferably 0.03 to 5 wt.-%, more preferably 0.05 to 2 wt.-%.

10. Perfumed product, comprising a mixture according to one of the claims 1 to 5 or, preferably, flavouring composition and/or aromatic substance composition according to one of the claims 7 to 9, preferably a perfume oil, in a sensorially active amount, wherein the proportion of the mixture or flavouring composition and/or aromatic substance composition related to the total weight of the product, preferably is in a range of 0.01 to 10 wt.-%, preferably in a range of 0.1 to 5 wt.-%, particularly preferably in a range of 0.25 to 3 wt.-%,
preferably wherein the product is selected from the group consisting of perfume extraits, eau de parfums, eau de toilettes, aftershaves, eau de colognes, pre-shave products, splash colognes, perfumed refreshing tissues, sour, alkaline and neutral cleaning agents, textile fresheners, ironing aids, liquid washing agents, powdery washing agents, laundry pretreatment agents, laundry conditioners, washing soaps, washing tablets, disinfectants, surface disinfectants, air improvers, aerosol sprays, waxes and polishes, personal care products, hand creams and lotions, foot creams and lotions, depilatory creams and lotions, aftershave creams and lotions, tanning creams and lotions, hair care products, deodorants and anti-transpirants, products of decorative cosmetics, candles, lamp oils, incense sticks, insecticides, repellants and propellants.

11. Method for producing a perfumed product, particularly a perfumed product according to claim 10, comprising the following steps:
i) Providing a mixture according to one of the claims 1 to 5 or a flavouring composition and/or aromatic substance composition according to one of the claims 7 to 9 or the substances of formula (Ia) 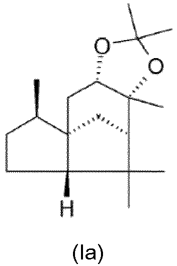 and the substances of formula (Ib), 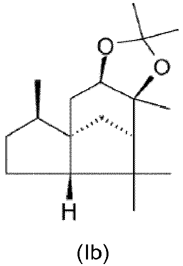 in a weight ratio as defined in claims 1 or 2,
ii) providing one or more further components of the perfumed product to be produced, and
iii) contacting or mixing the further components provided in step ii) with a sensorially active amount of the components provided in step i).

12. Use of a mixture according to one of the claims 1 to 5
(a) to mask or reduce the, one or more unpleasant olfactory impression(s) of one or more unpleasantly smelling substance(s),
and/or
(b) to enhance the, one or more pleasant olfactory impression(s) of one or more pleasantly smelling substance(s).

13. Use according to claim 12 in a composition, preferably a perfume oil, which comprises one or more (further) pleasantly and/or unpleasantly smelling substances, whose unpleasant olfactory impression is masked or reduced by the mixture according to one of the claims 1 to 5 and/or whose pleasant olfactory impression is enhanced by the mixture according to one of the claims 1 to 5, wherein this/these pleasantly and/or unpleasantly smelling substance or one, more or all of these pleasantly and/or unpleasantly smelling substances is/are selected from the group consisting of 3-(4-Methyl-1-cyclohex-3-enyl)-butanal, 4-(4-Hydroxyphenyl) butan-2-one, (E)-4-(2,6,6-Trimethyl-1-cyclohex-2-enyl)but-3-ene-2-one, (E)-4-(2,6,6-Trimethyl-1-cyclohex-2-enyl)but-3-ene-2-one, (E)-1-(2,6,6-trimethyl-cyclohexene-1-yl)pent-1-ene-3-one, (E)-4-[(1S)-1,2,6,6-tetramethylcyclohex-2-ene-1-yl]-but-3-ene-2-one, 1-(2,6,6-Trimethyl-1-cyclohex-2-enyl)pent-1-ene-3-one, [(Z)-Hex-3-enyl] methyl carbonate, 3-[(Z)-Hex-3-enoxy]propannitrile, 1-(2,3,8,8-Tetramethyl-1,3,4,5,6,7-hexa-hydronaphthalene-2-yl)ethanone, spiro[1,3-dioxolan-2,5'-(4',4',8',8'-tetramethyl-hexahydro-3',9'-methanonaphthalene)], [3R-(3α,3aβ,6β,7β,8aα)]-octa-hydro-6-methoxy-3,6,8,8-tetramethyl-1H-3a,7-methanoazulene, [3R-(3α,3aβ,7β,8aα)]-1-(2,3,4,7,8,8a-hexahydro-3,6,8,8-tetramethyl-1H-3a,7-methano-azulene-5-yl) ethane-1-one, 1-(2,2,6-Trimethylcyclohexyl) hexane-3-ol, 6,6-Dimethoxy-2,5,5-trimethylhex-2-ene, 2,6-Dimethyloct-7-ene-2-ol, 3,7-Dimethylocta-1,6-diene-3-ol, (3,7-Dimethylocta-1,6-diene-3-yl)acetate, (4-Methyl-1-propane-2-yl-1-cyclohex-2-enyl) acetate, (8E)-Cyclohexadec-8-ene-1-one, 16-Oxacyclohexa-decane-1-one, 1,3,4,6,7,8-Hexahydro-4,6,6,7,8,8-hexamethyl-cyclopenta(g)-2-benzopyrane, Ethoxymethoxy-cyclododecane, 1,1,2,3,3-Pentamethyl-2,5,6,7-tetrahydroinden-4-one, 1-(2,3,8,8-Tetramethyl-1,3,4,5,6,7-hexa-hydro-naphthalene-2-yl)ethanone.

14. Use according to claim 12 or 13 to enhance the or one or more olfactory impression(s) selected from the group consisting of the scents flowery, amber, woody, musk, violet, citrus and aldehyde.

15. Method
(a) for masking or reducing the, one or more unpleasant olfactory impression(s) of one or more unpleasantly smelling substances,
and/or
(b) for enhancing the, one or more pleasant olfactory impression(s) of one or more pleasantly smelling substances
comprising the following step:
Mixing the (a) pleasantly and/or (b) unpleasantly smelling substance(s) with a mixture according to one of the claims 1 to 5 or with a flavouring composition and/or aromatic substance composition according to one of the claims 7 to 9 or the substances of formula (Ia) 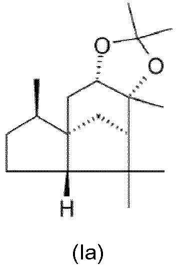 and the substances of formula (Ib) 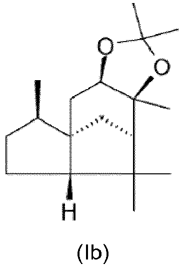 in a weight ratio as defined in claims 1 or 2,
wherein the amount of mixture according to one of the claims 1 to 5 or of the compound of formula (Ia) and the compound of formula (Ib) is sufficient to (a) enhance the pleasant olfactory impression(s) of the pleasantly smelling substance(s) and/or (b) to reduce or mask the unpleasant olfactory impression(s) of the unpleasantly smelling substance(s).

## Revendications

1. Mélange contenant ou se composant au moins pour l'essentiel de
- composé de formule (Ia) 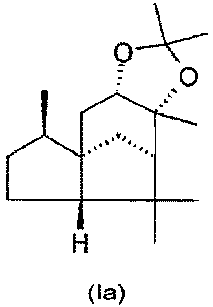 et de
- composé de formule (Ib), 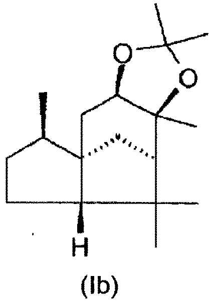
dans lequel le rapport pondéral du composé de formule (Ia) au composé de formule (Ib) dans le mélange se situe dans la plage allant de 90 : 10 à 99 : 1.

2. Mélange selon la revendication 1, dans lequel le rapport pondéral du composé de formule (la) au composé de formule (Ib) dans le mélange se situe dans la plage allant de 95 : 5 à 99 : 1, de préférence est de 98 : 2 à peu près,
et/ou dans lequel le mélange contient en outre un composé de formule (II) (cédralon), 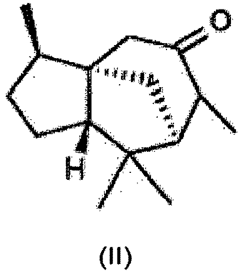 dans lequel le rapport pondéral de la quantité totale de composés des formules (Ia) et (Ib) à la quantité totale de composé de formule (II) dans le mélange est d'au moins 6 : 1, de préférence d'au moins 9 : 1, plus préférablement d'au moins 12 : 1, de manière particulièrement préférée d'au moins 25 : 1.

3. Mélange selon l'une quelconque des revendications 1 ou 2, dans lequel le mélange contient en outre un composé de formule (III) (cédrandiol), 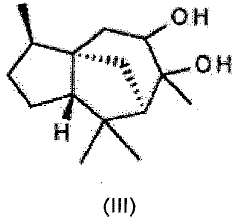 dans lequel le rapport pondéral de la quantité totale de composés des formules (Ia) et (Ib) à la quantité totale de composé de formule (III) dans le mélange est d'au moins 15 : 1, de préférence d'au moins 20 : 1, de manière particulièrement préférée d'au moins 25 : 1.

4. Mélange selon l'une quelconque des revendications 1 à 3, dans lequel au moins 90 % en poids de la quantité totale de composés des formules (Ia) et (Ib) dans le mélange se présentent sous forme cristalline.

5. Mélange selon l'une quelconque des revendications 1 à 4, apte à pouvoir être préparé par un procédé comprenant les étapes suivantes consistant à:
a) fournir un mélange d'isomères contenant ou se composant au moins pour l'essentiel de composés de formule (III) (cédrandiol), dans lequel le mélange d'isomères contient ou se compose au moins pour l'essentiel d'alpha,alpha-cédrandiol de formule (IIIa) 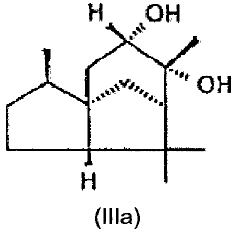 et de bêta,bêta-cédrandiol de formule (IIIb), 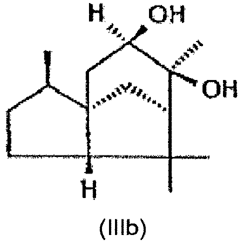 et dans lequel le rapport pondéral du composé de formule (IIIa) au composé de formule (IIIb) dans le mélange d'isomères se situe dans la plage allant de 95 : 5 à 99,9 : 0,1, de préférence d'au moins 98 : 2,
b) faire réagir le mélange d'isomères provenant de l'étape a) avec du diméthoxypropane en un rapport molaire d'au moins 1 : 2, par rapport à la quantité molaire totale de composés de formule (III) à la quantité molaire totale de diméthoxypropane,
le procédé comprenant de préférence en outre l'étape suivante consistant à:
c) faire cristalliser le produit de réaction provenant de l'étape b) à partir de solution alcoolique aqueuse.

6. Procédé de préparation d'un mélange contenant ou se composant au moins pour l'essentiel de
- composé de formule (Ia) 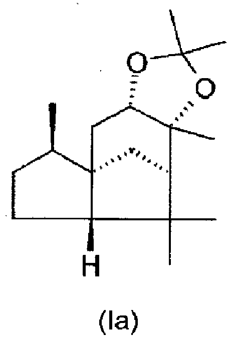 et de
- composé de formule (Ib), 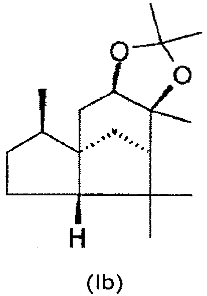
dans lequel le rapport pondéral du composé de formule (la) au composé de formule (Ib) dans le mélange se situe dans la plage allant de 90 : 10 à 99 : 1,
de préférence pour la préparation d'un mélange selon l'une quelconque des revendications 1 à 5,
comprenant les étapes suivantes consistant à:
a) fournir un mélange d'isomères contenant ou se composant au moins pour l'essentiel de composés de formule (III) (cédrandiol), dans lequel le mélange d'isomères contient ou se compose au moins pour l'essentiel d'alpha,alpha-cédrandiol de formule (IIIa) 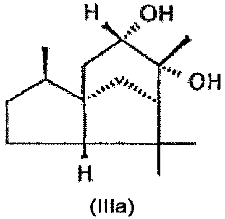 et de bêta,bêta-cédrandiol de formule (IIIb), 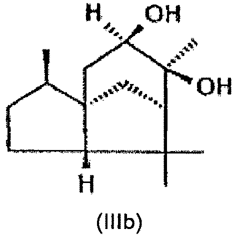 et dans lequel le rapport pondéral du composé de formule (IIIa) au composé de formule (IIIb) dans le mélange d'isomères se situe dans la plage allant de 95 : 5 à 99,9 : 0,1, de préférence est d'au moins 98 : 2,
b) faire réagir le mélange d'isomères provenant de l'étape a) avec du diméthoxypropane en un rapport molaire d'au moins 1 : 2, par rapport à la quantité molaire totale de composés de formule (III) à la quantité molaire totale de diméthoxypropane,
comprenant de préférence l'étape supplémentaire consistant à
c) faire cristalliser le produit de réaction provenant de l'étape b) à partir de solution alcoolique aqueuse.

7. Composition de substances odoriférantes et/ou aromatisantes, de préférence huile de parfum, contenant ou consistant en un mélange selon l'une quelconque des revendications 1 à 5, et de préférence en outre une ou plusieurs substances odoriférantes et/ou aromatisantes supplémentaires,
de préférence dans laquelle ladite substance odoriférante supplémentaire ou bien une, plusieurs ou l'ensemble des substances odoriférantes supplémentaires est ou bien sont choisie(s) dans le groupe se composant de 3-(4-méthyl-1-cyclohex-3-ényl)-butanal, 4-(4-hydroxyphényle) butan-2-one, (E)-4-(2,6,6-triméthyl-1-cyclohex-2-ényl)but-3-én-2-one, (E)-4-(2,6,6-triméthyl-1-cyclohex-2-ényl)but-3-én-2-one, (E)-1-(2,6,6-triméthyl-cyclohexén-1-yl)pent-1-én-3-one, (E)-4-[(1S)-1,2,6,6-tétraméthylcyclohex-2-én-1-yl]-but-3-én-2-one, 1-(2,6,6-triméthyl-1-cyclohex-2-ényl)pent-1-én-3-one, [(Z)-hex-3-ényl] méthyl carbonate, 3- [(Z)-hex-3-énoxy]propannitrile, 1-(2,3,8,8-tétraméthyl-1,3,4,5,6,7-hexa-hydronaphthalin-2-yl)éthanone, spiro[1,3-dioxolan-2,5'-(4',4',8',8'-tétraméthyl-hexahydro-3',9'-méthanonaphthaline)], [3R-(3α,3aβ,6β,7β,8aα)]-octa-hydro-6-méthoxy-3,6,8,8-tétraméthyl-1H-3a,7-miéthanoazulène, [3R-(3α,3aβ,7β,8aα)] -1-(2,3,4,7,8,8a-hexahydro-3,6,8,8-tétraméthyl-1H-3a,7-méthano-azulén-5-yl) éthan-1-one, 1-(2,2,6-triméthyl-cyclohexyl) hexan-3-ol, 6,6-diméthoxy-2,5,5-triméthylhex-2-ène, 2,6-diméthyloct-7-én-2-ol, 3,7-diméthylocta-1,6-dién-3-ol, (3,7-diméthylocta- 1,6-dién-3-yl)acétate, (4-méthyl-1-propan-2-yl-1-cyclohex-2-ényl) acétate, (8E)-cyclohexadéc-8-én-1-one, 16-oxacyclohexa-décan-1-one, 1,3,4,6,7,8-hexahydro- 4,6,6,7,8,8-hexaméthyl-cyclopenta(g)-2-benzopyrane, éthoxyméthoxy- cyclododécane, 1,1,2,3,3-pentaméthyl-2,5,6,7-tétrahydroindén-4-one, 1-(2,3,8,8-tétraméthyl-1,3,4,5,6,7-hexa-hydronaphthalin-2-yl)éthanone.

8. Composition de substances odoriférantes et/ou aromatisantes selon la revendication 7, dans laquelle la quantité de mélange selon l'une quelconque des revendications 1 à 5 ou bien la quantité de composé de formule (la) et de composé de formule (Ib) suffit
(a) pour masquer ou pour réduire la ou bien une ou plusieurs impressions olfactives désagréables d'une autre substance odoriférante et/ou aromatisante dans la composition de substances odoriférantes et/ou aromatisantes,
et/ou
(b) pour intensifier la ou bien une ou plusieurs impressions olfactives agréables d'une autre substance odoriférante et/ou aromatisante dans la composition de substances odoriférantes et/ou aromatisantes.

9. Composition de substances odoriférantes et/ou aromatisantes selon l'une quelconque des revendications 7 ou 8, de préférence huile de parfum, dans laquelle la quantité totale de composé de formule (Ia) et de composé de formule (Ib), par rapport au poids total de la composition de substances odoriférantes et/ou aromatisantes est comprise entre 0,01 et 10 % en poids, de préférence entre 0,03 et 5 % en poids, de manière particulièrement préférée entre 0,05 et 2 % en poids.

10. Produit parfumé contenant un mélange selon l'une quelconque des revendications 1 à 5 ou, de préférence, une composition de substances odoriférantes et/ou aromatisantes selon l'une quelconque des revendications 7 à 9, de préférence une huile de parfum, en une quantité sensoriellement efficace, dans lequel la proportion du mélange ou bien de la composition de substances odoriférantes et/ou aromatisantes par rapport au poids total du produit se situe de préférence dans la plage allant de 0,01 à 10 % en poids, de préférence dans la plage allant de 0,1 à 5 % en poids, de manière particulièrement préférée dans la plage allant de 0,25 à 3 % en poids,
préférence dans lequel le produit est choisi dans le groupe constitué par les extraits de parfum, les eaux de parfum, les eaux de toilette, les eaux après-rasage, les Eaux de Cologne, les produits de pré-rasage, les Splash Cologne, les lingettes rafraîchissantes parfumées, les agents de nettoyage acides, alcalins et neutres, les produits à rafraîchir les textiles, les aides au repassage, les détergents liquides, les détergents en poudre, les produits de prétraitement de linge, les adousissants de linge, les savons à laver, les pastilles lave-linge, les désinfectants, les désinfectants de surface, les assainisseurs d'air, les aérosols, les cires et polis, les produits de toilette, les crèmes et lotions pour les mains, les crèmes et lotions pour les pieds, les crèmes et lotions dépilatoires, les crèmes et lotions après-rasage, les crèmes et lotions de bronzage, les produits de soins des cheveux, les désodorisants et antiperspirants, les produits de la cosmétique décorative, les bougies, les huiles de lampe, les bâtonnets d'encens, les insecticides, les répulsifs et les carburants.

11. Procédé de production d'un produit parfumé, en particulier d'un produit parfumé selon la revendication 10, comprenant les étapes suivantes consistant à:
i) fournir un mélange selon l'une quelconque des revendications 1 à 5 ou une composition de substances odoriférantes et/ou aromatisantes selon l'une quelconque des revendications 7 à 9 ou les substances de formule (la) 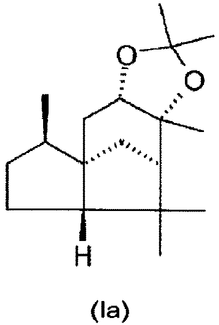 et de formule (Ib), 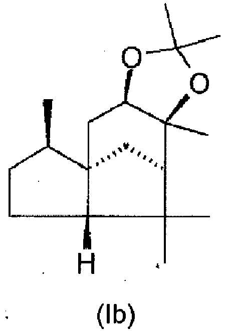 dans un rapport pondéral tel que défini dans la revendication 1 ou 2,
ii) fournir un ou plusieurs autres composants du produit parfumé à produire, et
iii) mettre en contact ou mélanger les autres composants fournis à l'étape ii), avec une quantité sensoriellement efficace des composants fournis à l'étape i).

12. Utilisation d'un mélange selon l'une quelconque des revendications 1 à 5
(a) pour masquer ou pour réduire la ou bien une ou plusieurs impressions olfactives désagréables d'une ou de plusieurs substance(s) ayant une odeur désagréable,
et/ou
(b) pour intensifier la ou bien une ou plusieurs impressions olfactives agréables d'une ou de plusieurs substance(s) ayant une odeur agréable.

13. Utilisation selon la revendication 12, dans une composition, de préférence une huile de parfum, contenant une ou plusieurs (autres) substances ayant une odeur agréable et/ou désagréable dont l'impression olfactive désagréable est masquée ou réduite par le mélange selon l'une quelconque des revendications 1 à 5 et/ou dont l'impression olfactive agréable est intensifiée par le mélange selon l'une quelconque des revendications 1 à 5, dans laquelle cette (ces) substance(s) ayant une odeur agréable et/ou désagréable ou bien une, plusieurs ou l'ensemble de ces substances ayant une odeur agréable et/ou désagréable est ou bien sont choisie(s) dans le groupe se composant de 3-(4-méthyl-1-cyclohex-3-ényl)-butanal, 4-(4-hydroxyphényle) butan-2-one, (E)-4-(2,6,6-triméthyl-1-cyclohex-2-ényl)but-3-én-2-one, (E)-4-(2,6,6-triméthyl-1-cyclohex-2-ényl)but-3-én-2-one, (E)-1-(2,6,6-triméthyl-cyclohexén-1-yl)pent-1-én-3-one, (E)-4-[(1S)-1,2,6,6-tétraméthylcyclohex-2-én-1-yl]-but-3-én-2-one, 1-(2,6,6-triméthyl-1-cyclohex-2-ényl)pent-1-én-3-one, [(Z)-hex-3-ényl] méthyl carbonate, 3- [(Z)-hex-3-énoxy]propannitrile, 1-(2,3,8,8-tétraméthyl-1,3,4,5,6,7-hexa-hydronaphthalin-2-yl)éthanone, spiro[1,3-dioxolan-2,5'-(4',4',8',8'-tétraméthyl-hexahydro-3',9'-méthanonaphthaline)], [3R-(3α,3aβ,6β,7β,8aα)]-octa-hydro-6-méthoxy-3,6,8,8-tétraméthyl-1H-3a,7-méthanoazulène, [3R-(3α,3aβ,7β,8aα)]-1-(2,3,4,7,8,8a-hexahydro-3,6,8,8-tétraméthyl-1H-3a,7-méthano-azulén-5-yl)éthan-1-one, 1-(2,2,6-triméthyl-cyclohexyl) hexan-3-ol, 6,6-diméthoxy-2,5,5-triméthylhex-2-ène, 2,6-diméthyloct-7-én-2-ol, 3,7-diméthylocta-1,6-dién-3-ol, (3,7-diméthylocta- 1,6-dién-3-yl)acétate, (4-méthyl-1-propan-2-yl-1-cyclohex-2-ényl) acétate, (8E)-cyclohexadéc-8-én-1-one, 16-oxacyclohexa-décan-1-one, 1,3,4,6,7,8-hexahydro-4,6,6,7,8,8-hexaméthyl-cyclopenta(g)-2-benzopyrane, éthoxyméthoxy-cyclododécane, 1,1,2,3,3-pentaméthyl-2,5,6,7-tétrahydroindén-4-one, 1-(2,3,8,8-tétraméthyl-1,3,4,5,6,7 -hexa-hydronaphthalin-2-yl)éthanone.

14. Utilisation selon la revendication 12 ou 13, pour intensifier la ou bien une ou plusieurs impressions olfactives choisies dans le groupe constitué par les notes olfactives fleurie, ambre, boisée, musc, violette, agrume et aldéhydique.

15. Procédé
(a) pour masquer ou pour réduire la ou bien une ou plusieurs impressions olfactives désagréables d'une ou de plusieurs substance(s) ayant une odeur désagréable,
et/ou
(b) pour intensifier la ou bien une ou plusieurs impressions olfactives agréables d'une ou de plusieurs substance(s) ayant une odeur agréable,
comprenant les étapes suivantes consistant à:
mélanger les substances ayant (a) une odeur agréable et/ou (b) une odeur désagréable avec un mélange selon l'une quelconque des revendications 1 à 5 ou une composition de substances odoriférantes et/ou aromatisantes selon l'une quelconque des revendications 7 à 9 ou les substances de formule (Ia) 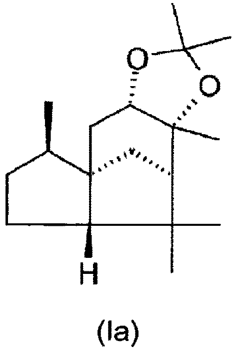 et de formule (Ib), 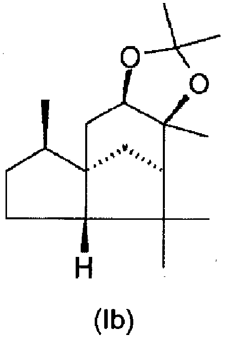 dans un rapport pondéral tel que défini dans la revendication 1 ou 2,
dans lequel la quantité de mélange selon l'une quelconque des revendications 1 à 5 ou bien de composé de formule (Ia) et de composé de formule (Ib) est suffisante pour (a) intensifier la ou bien les impressions olfactives agréables de la ou bien des substance(s) ayant une odeur agréable, et/ou pour (b) réduire ou masquer la ou bien les impressions olfactives désagréables de la ou bien des substance(s) ayant une odeur désagréable.
